# EUROPEAN PATENT SPECIFICATION

(11) **EP 2 279 790 B1**
(45) Date of publication and mention of the grant of the patent: **19.12.2012**
(21) Application number: 10275038.7
(22) Date of filing: 14.04.2010
(51) Int. Cl.: B01L 3/00, G01N 33/543, G01N 35/10, B01L 3/02, G01N 35/00

(54) **Sample Plate**
Probenplatte
Plaque d'échantillon

(30) Priority: 29.07.2009 GB 0913258; 07.10.2009 GB 0917555; 13.04.2010 GB 201006087
(43) Date of publication of application: 02.02.2011
(73) Proprietor: Dynex Technologies, Inc., Chantilly VA 20151-1621 (US)
(72) Inventor: Bunce, Adrian, Worthing, Sussex BN13 3DE (GB); Fusellier, Andrew, Guernsey, GY8 0PD (GB)
(74) Representative: Jeffrey, Philip Michael

(56) References cited:
- WO-A2-02/30561
- WO-A2-2004/111260
- US-A- 4 681 742
- US-A- 5 620 853
- US-A1- 2003 186 228
- US-A1- 2009 069 200

## Description

The present invention relates to a sample plate, a method of using a sample plate, a kit for performing an Enzyme Linked ImmunoSorbent Assay procedure, a kit for performing a nucleic acid probe procedure and a method of manufacturing a sample plate.

An automated reagent bead or microsphere dispenser for dispensing reagent beads or microspheres into a sample plate is disclosed. The sample plate may be used to carry out diagnostic testing such as Enzyme Linked ImmunoSorbent Assay ("ELISA") procedures or other immunoassay procedures. Alternatively, the sample plate may be used to carry out testing for DNA or RNA sequences.

Immunoassay procedures are a preferred way of testing biological products. These procedures exploit the ability of antibodies produced by the body to recognise and combine with specific antigens which may, for example, be associated with foreign bodies such as bacteria or viruses, or with other body products such as hormones. Once a specific antigen-antibody combination has occurred it can be detected using chromogenic, fluorescent or chemiluminescent materials or less preferably by using radioactive substances. Radioactive substances are less preferred due to environmental and safety concerns regarding their handling, storage and disposal. The same principles can be used to detect or determine any materials which can form specific binding pairs, for example using lectins, rheumatoid factor, protein A or nucleic acids as one of the binding partners.

ELISA is a particularly preferred form of immunoassay procedure wherein one member of the binding pair is linked to an insoluble carrier surface ("the solid phase") such as a sample vessel, and after reaction the bound pair is detected by use of a further specific binding agent conjugated to an enzyme ("the conjugate"). The procedures for ELISA are well known in the art and have been in use for both research and commercial purposes for many years. Numerous books and review articles describe the theory and practice of immunoassays. Advice is given, for example, on the characteristics and choice of solid phases for capture assays, on methods and reagents for coating solid phases with capture components, on the nature and choice of labels, and on methods for labelling components. An example of a standard textbook is "ELISA and Other Solid Phase Immunoassays, Theoretical and Practical Aspects", Editors D.M. Kemeny & S.J. Challacombe, published by John Wiley, 1988. Such advice may also be applied to assays for other specific binding pairs.

In the most common type of ELISA, the solid phase is coated with a member of the binding pair. An aliquot of the specimen to be examined is incubated with the solid coated solid phase and any analyte that may be present is captured onto the solid phase. After washing to remove residual specimen and any interfering materials it may contain, a second binding agent, specific for the analyte and conjugated to an enzyme is added to the solid phase. During a second incubation any analyte captured onto the solid phase will combine with the conjugate. After a second washing to remove any unbound conjugate, a chromogenic substrate for the enzyme is added to the solid phase. Any enzyme present will begin to convert the substrate to a chromophoric product. After a specified time the amount of product formed may be measured using a spectrophotometer, either directly or after stopping the reaction.

It will be realised that the above is an outline description of a general procedure for bioassay and that many variants are known in the art including fluorogenic and luminogenic substrates for ELISA, direct labelling of the second member of the binding pair with a fluorescent or luminescent molecule (in which case the procedure is not called an ELISA but the process steps are very similar) and nucleic acids or other specific pairing agents instead of antibodies as the binding agent. However, all assays require that fluid samples, e.g. blood, serum, urine, etc., are aspirated from a sample tube and are then dispensed into a solid phase. Samples may be diluted prior to being dispensed into the solid phase or they may be dispensed into deep well microplates, diluted in situ and then the diluted analyte transferred to the functional solid phase.

The most common type of solid phase is a standard sample vessel known as a microplate which can be stored easily and which may be used with a variety of biological specimens. Microplates have been available commercially since the 1960s and are made from e.g. polystyrene, PVC, Perspex or Lucite and measure approximately 5 inches (12.7 cm) in length, 3.3 inches (8.5 cm) in width, and 0.55 inches (1.4 cm) in depth. Microplates made from polystyrene are particularly preferred on account of polystyrene's enhanced optical clarity which assists visual interpretation of the results of any reaction. Polystyrene microplates are also compact, lightweight and easily washable. Microplates manufactured by the Applicants are sold under the name "MICROTITRE" (RTM). Known microplates comprise 96 wells (also commonly known as "microwells") which are symmetrically arranged in an 8 x 12 array. Microwells typically have a maximum volume capacity of approximately 350 µl. However, normally only 10-200 µl of fluid is dispensed into a microwell. In some arrangements of the microplate the microwells may be arranged in strips of 8 or 12 wells that can be moved and combined in a carrier to give a complete plate having conventional dimensions.

Positive and negative controls are generally supplied with commercial kits and are used for quality control and to provide a relative cut-off. After reading the processed microplate, the results of the controls are checked against the manufacturer's validated values to ensure that the analysis has operated correctly and then the value is used to distinguish positive from negative specimens and a cut-off value is calculated. Standards are usually provided for quantitative assays and are used to build a standard curve from which the concentration of analyte in a specimen may be interpolated.

It will be recognised that the ELISA procedure as outlined above involves multiple steps including pipetting, incubation, washing, transferring microplates between activities, reading and data analysis. In recent years systems have been developed which automate the steps (or "phases") involved in the ELISA procedures such as sample distribution, dilution, incubation at specific temperatures, washing, enzyme conjugate addition, reagent addition, reaction stopping and the analysis of results. The pipette mechanism used to aspirate and dispense fluid samples uses disposable tips which are ejected after being used so as to prevent cross-contamination of patients' samples. Multiple instrumental controls are in place to ensure that appropriate volumes, times, wavelengths and temperatures are employed, data transfer and analysis is fully validated and monitored. Automated immunoassay apparatus for carrying out ELISA procedures are now widely used in laboratories of e.g. pharmaceutical companies, veterinary and botanical laboratories, hospitals and universities for in-vitro diagnostic applications such as testing for diseases and infection, and for assisting in the production of new vaccines and drugs.

ELISA kits are commercially available which consist of microplates having microwells which have been coated by the manufacturer with a specific antibody (or antigen). For example, in the case of a hepatitis B antigen diagnostic kit, the kit manufacturer will dispense anti-hepatitis B antibodies which have been suspended in a fluid into the microwells of a microplate. The microplate is then incubated for a period of time, during which time the antibodies adhere to the walls of the microwells up to the fluid fill level (typically about half the maximum fluid capacity of the microwell). The microwells are then washed leaving a microplate having microwells whose walls are uniformly covered with anti-hepatitis B antibodies up to the fluid fill level.

A testing laboratory will receive a number of sample tubes containing, for example, body fluid from a number of patients. A specified amount of fluid is then aspirated out of the sample tube using a pipette mechanism and is then dispensed into one or more microwells of a microplate which has been previously prepared by the manufacturer as discussed above. If it is desired to test a patient for a number of different diseases then fluid from the patient must be dispensed into a number of separate microplates, each coated by its manufacturer with a different binding agent. Each microplate can then be processed separately to detect the presence of a different disease. It will be seen that to analyse several different analytes requires a multiplicity of microplates and transfer of aliquots of the same specimen to the different microplates. This leads to large numbers of processing steps and incubators and washing stations that can cope with many microplates virtually simultaneously. In automated systems this requires instruments to have multiple incubators and complex programming is required to avoid clashes between microplates with different requirements. For manual operation either several technicians are required or the throughput of specimens is slow. It is possible to combine strips of differently coated microwells into a single carrier, add aliquots of a single specimen to the different types of well and then perform the ELISA in this combined microplate. Constraints on assay development, however, make this combination difficult to achieve and it is known in the art that for users to combine strips in this fashion can lead to errors of assignment of result, while manufacture of microplates with several different coatings in different microwells presents difficulties of quality control.

Conventional ELISA techniques have concentrated upon performing the same single test upon a plurality of patient samples per microplate or in detecting the presence of one or more of a multiplicity of analytes in those patients without distinguishing which of the possible analytes is actually present. For example, it is commonplace to determine in a single microwell whether a patient has antibodies to HIV-1 or HIV-2, or HIV-1 or -2 antigens, without determining which analyte is present and similarly for HCV antibodies and antigens.

However, a new generation of assays are being developed which enable multiplexing to be performed. Multiplexing enables multiple different tests to be performed simultaneously upon the same patient sample.

A recent approach to multiplexing is to provide a microplate comprising 96 sample wells wherein an array of different capture antibodies is disposed in each sample well. The array comprises an array of 20 nl spots each having a diameter of 350 µm. The spots are arranged with a pitch spacing of 650 µm. Each spot corresponds with a different capture antibody.

Multiplexing enables a greater number of data points and more information per assay to be obtained compared with conventional ELISA techniques wherein each sample plate tests for a single analyte of interest. The ability to be able to combine multiple separate tests into the same assay can lead to considerable time and cost savings. Multiplexing also enables the overall footprint of the automated apparatus to be reduced.

Although there are many advantageous aspects to current known ELISA techniques and to the new multiplex techniques which are currently being developed, it is nonetheless desired to provide a sample plate and associated automated apparatus which has an improved format and which provides a greater flexibility than state of the art ELISA arrangements.

In addition to ELISA procedures it is also known to use a hybridization probe to test for the presence of DNA or RNA sequences. A hybridization probe typically comprises a fragment of DNA or RNA which is used to detect the presence of nucleotide sequences which are complementary to the DNA or RNA sequence on the probe. The hybridization probe hybridizes to single-stranded nucleic acid (e.g. DNA or RNA) whose base sequence allows pairing due to complementarity between the hybridization probe and the sample being analysed. The hybridization probe may be tagged or labelled with a molecular marker such as a radioactive or more preferably a fluorescent molecule. The probes are inactive until hybridization at which point there is a conformational change and the molecule complex becomes active and will then fluoresce (which can be detected under UV light) DNA sequences or RNA transcripts which have a moderate to high sequence similarity to the probe are then detected by visualising the probe under UV light.

US 2009/0069200 discloses a system for preparing arrays of biomolecules.

An assay device and assembly for detecting an analyte in a liquid sample is disclosed in US-5620853 (Chiron Corporation). The assay device comprises a moulded well comprising fingers which protrude up from the bottom of the well and into which a reagent bead is dispensed. The reagent bead is captured in the fingers but can still move up and down within the finger height. The assay device is arranged to expose the reagent bead to as much fluid flow as possible and to rely upon signal from the underside of the reagent bead to produce results.

There are a number of problems with the arrangement disclosed in US-5620853.

Firstly, since the reagent beads are free to move up and down within the finger height then it is possible that a reagent bead may become stuck at an undesired height during a processing or reading step. In particular, the design of the well is relatively intricate and complex and any movement of, or damage to, the fingers could result in a reagent bead becoming stuck at an undesired height. The fingers also protrude from the base which makes them susceptible to damage particularly during pipetting and washing stages. If a reagent bead does become stuck at an undesired height within the fingers then this is highly likely to have an adverse effect upon the accuracy of the testing procedures.

Secondly, the design of the well with fingers which are arranged to receive a single reagent bead is such that fluid is pipetted next to the bead and the bead is covered by the rising fluid in the well. The single wells needs approximately 300 µl of fluid. US-5620853 also discloses an arrangement wherein multiple wells are in fluid communication with each other. For the multi-well arrangement, each well will need approximately 300 µl of fluid. It will be apparent, therefore, that the multi-well arrangement requires an excessive amount of fluid to be dispensed relative to conventional systems.

Thirdly, the arrangement of fingers reduces the maximum packing density of wells for a given size sample plate so that relatively few tests can be performed on a given sample plate.

Fourthly, the multi-well arrangement disclosed in US-5620853 is particularly prone to crosstalk.

Fifthly, the arrangement disclosed in US-5620853 is such that when a single bead is used then the homogeneity of the fluid is only affected by the protruding fingers. There are likely to be regions of the well which will trap unmixed fluid. The multi-well arrangement also suffers from the serious problem that any fluid required to go over all beads has to pass through a tortuous path to get from one well to another. This will cause serious problems in terms of fluid mixing and bead to bead repeatability. The single well arrangement is completely different to the in-line multi-well arrangement disclosed in US-5620853 and the two different arrangements would therefore have quite different fluid characteristics. This is likely to result in different fluid behaviours depending upon the arrangement used and hence there is likely to be significant variation in results depending upon whether a single well or a multi-well format was used. Although in theory the two different arrangements could be validated independently, this would result in increased cost and reduced throughput.

Finally, the sample well disclosed in US-5620853 is relatively complex to manufacture and is likely to suffer from unreliability issues during manufacture. The long thin fingers are difficult to form by moulding and would be prone to damage during manufacture or during use. The fingers also have a feature at the top which in a mould tool would be an undercut. When the part is ejected off the tool the fingers must bend for the feature to get past the tool material. Such a manufacturing process is generally undesirable due to unreliability issues. Furthermore, any change in the process parameters is likely to affect the ability to release the part from the tool and leave the part intact to the correct mechanical tolerances. The position of the fingers relative to each other would be critical to allow the reagent bead to move up and down correctly and also to ensure that the reagent bead does not come out of the top of the fingers. This would be very difficult, in practice, to control in a mass production environment. It is also noted that the design of the single bead arrangement is completely different to the design of the multi-well arrangement. As a result, completely different tool designs would be required which again would greatly increase the complexity of manufacture. In a high volume manufacturing environment the combination of the design features and quality assurance concerns would make the sample plates excessively expensive to produce.

It is therefore desired to provide an improved sample plate for retaining reagent beads.

According to an aspect of the present invention there is provided a sample plate as claimed in claim 1.

The sample plate according to the present invention is particularly advantageous compared to the sample plate disclosed in US-5620853.

Reagent beads are inserted into a sample plate having a plurality of tapered holes or sections which act to firmly secure or lock the reagent beads in position once inserted. A preset force is preferably used to insert the reagent beads. The sample plate according to the present invention is therefore particularly robust during manufacture and in subsequent processing stages including the stage of inserting reagent beads into the tapered holes and subsequent handling and processing of the sample plate. Once the reagent beads have been inserted into a sample plate then they are not free to move in any direction and essentially become a fixed part of the sample plate. The angle of the taper is preferably arranged so that reagent beads are locked or are otherwise firmly secured into the holes making the arrangement very reliable.

According to the preferred embodiment the reagent beads are preferably opaque and signal is preferably only taken from the top of the bead. The bottom of the bead below the press fit line does not, preferably, come into contact with the fluid. A sample plate with inserted reagent beads therefore resembles fairly closely an empty conventional sample well. According to the preferred embodiment the reagent beads do not protrude above the bottom of the sample well and so are preferably not susceptible to damage through handling, pipetting or washing. However, less preferred embodiments are also contemplated wherein one or more of the reagent beads may protrude slightly above the bottom of the sample well.

An advantageous aspect of the preferred embodiment is that since the reagent beads are preferably arranged to be inserted so that they are flush with the bottom of the well then the sample plate according to the present invention can be used with known automated microplate processing systems without requiring any hardware modifications. Furthermore, the sample well according to the preferred embodiment is essentially a cylinder having proportions which are similar to that of a well of a conventional microplate so the fluid and other handling characteristics of the sample well are well known. Processing steps according to the preferred embodiment such as pipetting, mixing, washing and incubation preferably follow the same type of fluid characteristics that conventional microplates go through.

The sample plate according to the preferred embodiment preferably has a fluid capacity of approximately 800 µl but advantageously, in use, only approximately 300 µl of fluid is required in order to cover all the reagent beads disposed in the base of the sample plate.

Another advantageous feature of the sample plate according to the preferred embodiment is that fluid can be dispensed directly into the centre of the sample well and according to the preferred embodiment the sample plate may be arranged so that the pockets, recesses or bores for securing reagent beads are not arranged in the central region of the sample well. Such an arrangement is particularly advantageous in that reagent which preferably coats the reagent beads is not inadvertently washed off the reagent beads by the force of the fluid jet from a wash head or pipette tip.

The sample plate according to the preferred embodiment preferably enables multiple tests to be carried out in a single sample well. This is achieved by inserting different reagent beads into separate bores in the same sample well thereby enabling multiplexing to be performed. According to the preferred embodiment reagent beads can be pressed into the tapered holes in the base of the well as desired which results in a high degree of flexibility and the ability to use the entire sample well with a high efficiency.

A sample plate according to an embodiment of the present invention may comprise one or more 12 mm diameter sample wells. Each sample well may have a cross sectional surface area of 58 mm² and in total 54 sample wells of this size can be fitted into a conventional microplate footprint. Within each sample well a varied number of beads can be inserted. The tapered bores can have different diameters to accommodate different size reagent beads if desired.

According to other embodiments one or more sample wells may comprise 6 x 3.0 mm diameter pockets, recesses or tapered bores, 10 x 2.0 mm diameter pockets, recesses or tapered bores or 21 x 1.75 mm pockets, recesses or tapered bores. The central region of the sample well is preferably kept free of pockets, recesses or tapered bores. The pockets, recesses or tapered bores may be arranged in one or more concentric circles or other patterns about the central region of the sample well.

According to an embodiment a sample plate having an array of 9 x 6 sample wells may be provided. If 6 pockets, recesses or tapered bores are provided per sample well, then the sample plate can accommodate 324 reagent beads per plate. If 10 pockets, recesses or tapered bores are provided per sample well, then the sample plate can accommodate 540 reagent beads per plate. If 21 pockets, recesses or tapered bores are provided per sample well, then the sample plate can accommodate 1134 reagent beads per plate.

According to the preferred embodiment the sample plate according to the preferred embodiment does not suffer from fluid mixing problems. The sample wells comprise beads which are pressed or inserted into the pockets, recesses or tapered bores. The tops of the reagent beads once inserted are preferably flush or level with the bottom of the sample well. According to the preferred embodiment the mixing is using fluid that is above the surface of the beads to pull fluid from the pocket area around the bead.

A further advantageous aspect of the present invention is that the sample plate according to the present invention is relatively simple to manufacture compared with other known arrangements. The sample plate can be manufactured by moulding using an open and shut tool so that the manufacturability is high and reliable. The injection mould tool design used to formed the sample plates is simple and does not require the use of undercuts or thin features to mould. As a result, the production of sample plates having different formats can be readily achieved. A tool that produces a sample well with 6 pockets or bores can be readily adapted to produce a sample well having a different number (e.g. 21) of pockets.

Another advantage of the preferred embodiment is that validation of different well designs and formats can be achieved simply since the test protocols can remain essentially the same. Pipetting and incubation would not change and the washing procedure would only require, at most, a minor alteration to the aspirate routine.

It is apparent, therefore, that the sample plate according to the present invention is particularly advantageous compared to other known sample plates including the sample plate disclosed in US-5620853.

The tapered section preferably has a taper selected from the group consisting of: (i) 2-4°; (ii) 4-6°; (iii) 6-8°; and (iv) 8-10°.

The one or more pockets or recesses may comprise a countersunk or enlarged portion for facilitating the insertion of a reagent bead or microsphere into one or more of the pockets or recesses.

The one or more sample wells preferably comprise at least 1, 2, 3, 4, 5, 6, 7, 8, 9, 10, 11, 12, 13, 14, 15, 16, 17, 18, 19 or 20 pockets or recesses each comprising a bore having a tapered section and which are each arranged and adapted to receive, in use, a reagent bead or microsphere.

The one or more pockets, recesses or bores provided in the base portion are preferably arranged: (i) circumferentially around a central portion of the sample well; and/or (ii) with a plurality of pockets or recesses arranged circumferentially around one more central pockets or recesses; and/or (iii) in a substantially close-packed manner; and/or (iv) in a substantially symmetrical or asymmetrical manner; and/or (v) in a substantially linear or curved manner; and/or (vi) in a substantially regular or irregular manner; and/or (vii) in an array; and/or (viii) in one or more concentric circles with no pocket, recess or bore located at the centre of the base portion.

The sample plate is preferably fabricated or otherwise made from polystyrene.

The sample plate may comprise either a strip or an array format. For example, according to a preferred embodiment the sample plate may comprise a 6x1 strip. According to another preferred embodiment the sample plate may comprise a 9x6 strip.

According to other embodiments the sample plate may comprise sample wells arranged in an A x B format wherein:
A is selected from the group consisting of: (i) 1; (ii) 2; (iii) 3; (iv) 4; (v) 5; (vi) 6; (vii) 7; (viii) 8; (ix) 9; (x) 10; and (xi) > 10; and
B is selected from the group consisting of: (i) 1; (ii) 2; (iii) 3; (iv) 4; (v) 5; (vi) 6; (vii) 7; (viii) 8; (ix) 9; (x) 10; and (xi) > 10.

According to an embodiment one or more of the sample wells may be interconnected to one or more other sample wells by one or more frangible regions or connections so that the sample plate can be separated by a user into a plurality of smaller sample plates. This enables a sample plate to be snapped or broken into a plurality of smaller sample plates. For example, a 6x1 strip of sample plates may be snapped into individual 1x1 sample plates comprising a single sample well or into two sample plates each comprising a 3x1 strip of sample wells.

The sample plate may comprise an Immunoassay sample plate. Alternatively, the sample plate may comprise a hybridization probe for detecting the presence of complementary DNA or RNA samples.

The sample plate preferably comprises a base having a female, male or other docking portion for securing the sample plate to a corresponding male, female or other docking portion of a plate frame holder.

At least some or substantially all of the reagent beads or microspheres preferably carry, comprise or are otherwise coated with a reagent, wherein the reagent is arranged and adapted to assay for an analyte of interest in a sample liquid.

According to an alternative embodiment at least some or substantially all of the reagent beads or microspheres carry, comprise or are otherwise coated with a nucleic acid probe, wherein the nucleic acid probe is arranged and adapted to hybridize with single-stranded nucleic acid, DNA or RNA.

According to another arrangement there is provided a combination of a plate frame holder and a sample plate as disclosed above.

The plate frame holder preferably comprises a male, female or other docking portion for firmly securing the sample plate to the plate frame holder.

According to an aspect of the present invention there is provided a method of using a sample plate to analyse a sample for multiple analytes as claimed in claim 8.

According to an aspect of the present invention there is provided a kit for performing an Enzyme Linked ImmunoSorbent Assay (ELISA) procedure as claimed in claim 9.

According to another aspect of the present invention there is provided a kit for performing a nucleic acid probe procedure as claimed in claim 10.

According to an aspect of the present invention there is provided a method of manufacturing a sample plate as claimed in claim 11.

According to an embodiment one or more of the sample wells have a diameter or maximum width selected from the group consisting of: (i) < 1 mm; (ii) 1-2 mm; (iii) 2-3 mm; (iv) 3-4 mm; (v) 4-5 mm; (vi) 5-6 mm; (vii) 6-7 mm; (viii) 7-8 mm; (ix) 8-9 mm; (x) 9-10 mm; (xi) 10-11 mm; (xii) 11-12 mm; (xiii) 12-13 mm; (xiv) 13-14 mm; (xv) 14-15 mm; (xvi) 15-16 mm; (xvii) 16-17 mm; (xviii) 17-18 mm; (xix) 18-19 mm; (xx) 19-20 mm; and (xxi) > 20 mm.

The sample well may comprise one or more circular, oblong, triangular, square, rectangular, pentagonal, hexagonal, septagonal, octagonal, nonagonal, decagonal or polygonal fluid receiving areas.

The reagent beads or microspheres which are dispensed, in use, into one or more of the pockets, recesses or bores preferably have a diameter selected from the group consisting of: (i) 0.5-1.0 mm; (ii) 1.0-1.5 mm; (iii) 1.5-2.0 mm; (iv) 2.0-2.5 mm; (v) 2.5-3.0 mm; (vi) 3.0-3.5 mm; (vii) 3.5-4.0 mm; (viii) 4.0-4.5 mm; (ix) 4.5-5.0 mm; and (x) > 5.0 mm.

At least some or substantially all of the reagent beads or microspheres which are dispensed, in use, into one or more of the pockets, recesses or bores may carry or comprise a reagent, wherein the reagent is arranged and adapted: (i) to analyse samples; and/or (ii) to analyse samples by nucleic acid amplification reactions; and/or (iii) to analyse samples by polymerase chain reactions (PCR); and/or (iv) to analyse samples by an immunoassay process; and/or (v) to analyse samples by using a hybridization probe technique.

At least some or substantially all of the reagent beads or microspheres which are dispensed, in use, into one or more of the pockets, recesses or bores comprise polystyrene, plastic or a polymer.

According to an embodiment at least some or substantiallly all of the reagent beads or microspheres which are dispensed, in use, into one or more of the pockets, recesses or bores comprise a ferrous or magnetic coating or have a ferrous or magnetic property.

At least some or substantially all of the reagent beads or microspheres which are dispensed, in use, into one or more of the pockets, recesses or bores preferably comprise an anti-static coating or have an anti-static property.

According to an arrangement the one or more of the reagent bead or microsphere dispensers comprise a tube containing, in use, a plurality of reagent beads or microspheres.

One or more of the reagent bead or microsphere dispensers may comprise a helical screw, an auger or a reagent bead or microsphere transmission device for passing or transmitting one or more reagent beads or microspheres contained within the reagent bead or microsphere dispenser to a dispensing region, dispensing end or dispensing tip of the reagent bead or microsphere dispenser.

The apparatus may further comprise one or more sensors for sensing whether or not one or more reagents beads have been dispensed from one or more of the reagent bead or microsphere dispensers.

The apparatus may further comprise a translation stage for moving the sample plate relative to one or more reagent bead or microsphere dispensers.

The apparatus may further comprise a fluid dispensing device for dispensing fluid into one or more of the fluid receiving areas of one or more sample wells.

The fluid dispensing device is preferably arranged and adapted to dispense x ml of fluid at a time into the one or more fluid receiving areas of one or more sample wells, wherein x is preferably selected from the group consisting of: (i) < 10; (ii) 10-20; (iii) 20-30; (iv) 30-40; (v) 40-50; (vi) 50-60; (vii) 60-70; (viii) 70-80; (ix) 80-90; (x) 90-100; (xi) 100-110; (xii) 110-120; (xiii) 120-130; (xiv) 130-140; (xv) 140-150; (xvi) 150-160; (xvii) 160-170; (xviii) 170-180; (xix) 180-190; (xx) 190-200; and (xxi) > 200.

According to an embodiment the apparatus further comprises an image analysis device or camera for determining whether or not a reagent bead or microsphere has been dispensed or is otherwise present in a pocket, recess or bore.

The sample plate preferably has a first colour and the reagent beads or microspheres preferably have a second different colour which contrasts with the first colour in order to facilitate visual detection of the presence or absence of a reagent bead or microsphere in a pocket, recess or bore.

According to an embodiment the sample plate may further comprise a luminescence or fluorescence marker.

The apparatus may further comprise a luminescence or fluorescence detecting device for determining whether or not a reagent bead or microsphere has been dispensed or is otherwise present in a pocket, recess or bore by determining whether or not a reagent bead or microsphere obstructs or partially obstructs the luminescence or fluorescence marker.

The apparatus preferably further comprises a magnetic and/or electrical and/or capacitive and/or mechanical sensor for sensing whether or not a reagent bead or microsphere has been dispensed or is otherwise present in a pocket, recess or bore of a sample plate.

The control system preferably determines the number of reagent beads or microspheres present and/or the number of reagent beads or microspheres absent and/or the number of reagent beads or microspheres dispensed and/or the number of reagent beads or microspheres desired to be dispensed in a sample well.

According to an embodiment the control system measures and/or adjusts the volume of fluid dispensed or desired to be dispensed into a sample well dependent upon the number of reagent beads or microspheres determined to be present and/or absent and/or dispensed and/or desired to be dispensed in the sample well.

The control system is preferably arranged and adapted to ensure that at least some or substantially all reagent beads or microspheres in a sample well are at least partially or fully immersed by a fluid when the fluid is dispensed into the sample well.

The control system is preferably arranged and adapted to ensure that the height of fluid dispensed into a sample well remains substantially constant irrespective of the number of reagent beads or microspheres present, absent, dispensed or desired to be dispensed into the sample well.

The one or more of the sample wells preferably have a diameter or maximum width selected from the group consisting of: (i) < 1 mm; (ii) 1-2 mm; (iii) 2-3 mm; (iv) 3-4 mm; (v) 4-5 mm; (vi) 5-6 mm; (vii) 6-7 mm; (viii) 7-8 mm; (ix) 8-9 mm; (x) 9-10 mm; (xi) 10-11 mm; (xii) 11-12 mm; (xiii) 12-13 mm; (xiv) 13-14 mm; (xv) 14-15 mm; (xvi) 15-16 mm; (xvii) 16-17 mm; (xviii) 17-18 mm; (xix) 18-19 mm; (xx) 19-20 mm; and (xxi) > 20 mm.

The sample well preferably comprises one or more circular, oblong, triangular, square, rectangular, pentagonal, hexagonal, septagonal, octagonal, nonagonal, decagonal or polygonal fluid receiving areas.

Various embodiments of the present invention will now be described together with other arrangements given for illustrative purposes only, by way of example only, and with reference to the accompanying drawings in which:
Fig. 1 shows an arrangement wherein a plurality of reagent bead or microsphere dispensers are attached to a rotatable carousel and a sample plate is mounted on a translation stage below an arm which extends from the rotatable carousel and which is engaged with a reagent bead or microsphere dispenser;
Fig. 2 shows a reagent bead or microsphere dispenser;
Fig. 3 shows in greater detail a plurality of reagent bead or microsphere dispensers mounted to a carousel and an arm in engagement with a reagent bead or microsphere dispenser;
Fig. 4A shows a first configuration of a sample well of a sample plate not in accordance with the present invention, Fig. 4B shows a second different configuration of a sample well of a sample plate not in accordance with the present invention and Fig. 4C illustrates how different species or types of reagent beads or microspheres may be dispensed into different reagent bead or microsphere receiving chambers or sections of a sample well of a sample plate not in accordance with the present invention;
Fig. 5 shows a strip of sample wells not in accordance with the present invention;
Fig. 6 shows a sample well of a sample plate according to a preferred embodiment of the present invention;
Fig. 7A shows a plan view of a sample well of a sample plate according to the preferred embodiment, Fig. 7B shows in greater detail the bottom of a sample well according to the preferred embodiment and Fig. 7C shows a reagent bead or microsphere dispensed in a pocket of a sample well according to the preferred embodiment;
Fig. 8A shows a reagent bead or microsphere dispenser and Fig. 8B shows a cutaway view of the reagent bead or microsphere dispenser;
Fig. 9 shows an exploded view of the reagent bead or microsphere;
Fig. 10 shows a microarrayer comprising a reagent bead or microsphere syringe pick-up device mounted on an x-y-z translation stage and engaged with a reagent bead or microsphere dispenser above a sample plate;
Fig. 11 shows in greater detail a cutaway view of a reagent bead or microsphere syringe pick-up device attached to a reagent bead or microsphere dispenser;
Fig. 12A shows a reagent bead or microsphere dispenser being transported by a reagent bead or microsphere syringe pick-up device and Fig. 12B shows a reagent bead or microsphere in the process of being dispensed from a reagent bead or microsphere dispenser by a plunger mechanism which is actuated by the reagent bead or microsphere syringe pick-up device;
Fig. 13A shows a reagent bead or microsphere syringe in the process of being ejected from the reagent bead or microsphere syringe pick-up device and Fig. 13B shows the reagent bead or microsphere syringe having been ejected from the reagent bead or microsphere pick-up device;
Fig. 14A shows nine sample plates loaded into a plate frame, wherein each sample plate comprises a strip of 6 sample wells and Fig. 14B shows a plate frame into which sample plate one or more sample plates may be loaded;
Fig. 15A shows in greater detail a strip of six sample wells and Fig. 15B shows a strip of six sample wells being loaded into a plate frame;
Fig. 16A shows a single well being loaded into a plate frame, Fig. 16B shows in greater detail two sample wells connected by a break apart feature, Fig. 16C shows a sample well having an end feature and Fig. 16D shows a sample well having an ID and orientation tab;
Fig. 17A shows the underneath of a strip of sample wells, Fig. 17B shows a female alignment and retaining feature which helps to align a strip of sample wells with a plate frame and Fig. 17C shows a corresponding male alignment and retaining feature which is provided in the base of the plate frame; and
Fig. 18 shows a cross-sectional view of a strip of sample wells and shows that according to the preferred embodiment the sample wells have a plurality of tapered bores wherein the angle of the taper is 6.0°.

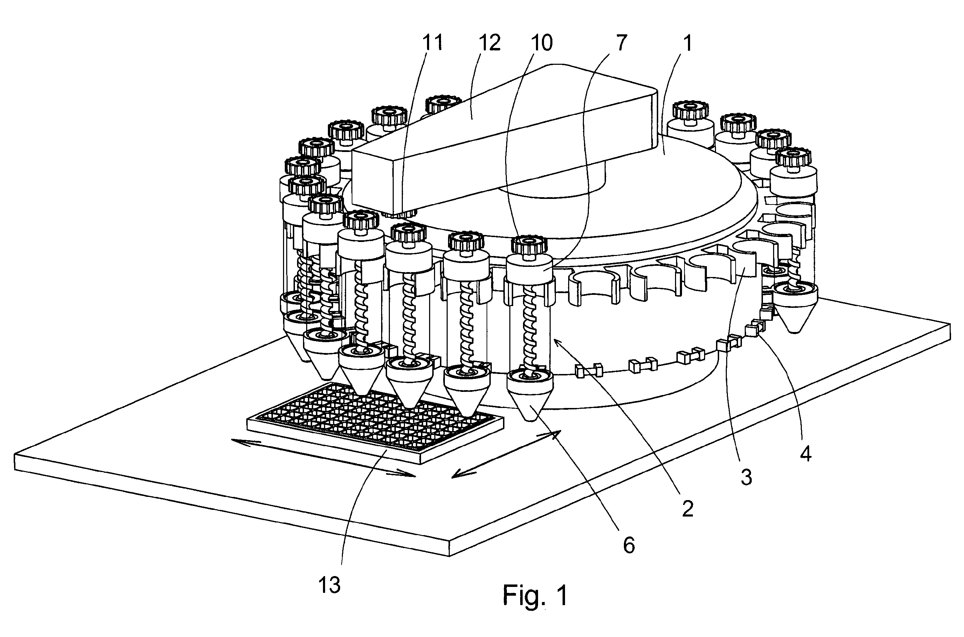

An illustrative arrangement will now be described in greater detail with reference to Fig. 1. A rotatable carousel 1 is provided which comprises a plurality of docking portions or sections disposed around the outer circumference or perimeter of the carousel 1. According to the particular arrangement shown in Fig. 1 twenty-four docking portions are provided although other arrangements are contemplated wherein a different number of docking portions may be provided. For example, according to other arrangements 1, 2, 3, 4, 5, 8, 7, 8, 9, 10, 11, 12, 13, 14, 15, 16, 17, 18, 19, 20, 21, 22, 23, 25, 26, 27, 28, 29, 30 or > 30 docking portions may be provided.

A plurality of reagent bead or microsphere dispensers 2 are attached or are otherwise secured in use to the carousel 1 at some or all of the docking portions. Each docking portion comprises an upper clip 3 and a lower retaining pin 4. The upper clip 3 and lower retaining pin 4 are used to secure a reagent bead or microsphere dispenser 2 to the docking portion. Other arrangements are contemplated wherein the retaining pin 4 may be provided in an upper position and the clip 3 may be provided in a lower position.

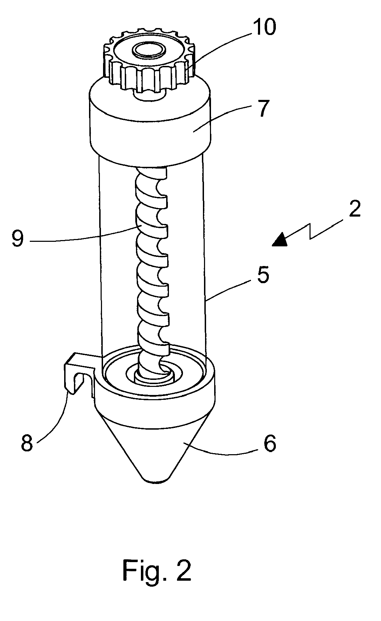

A single reagent bead or microsphere dispenser 2 is shown in greater detail in Fig. 2. The reagent bead or microsphere dispenser 2 comprise a tubular body 5 having a lower funnel-shaped dispensing portion 6 and an upper cap portion 7. Each reagent bead or microsphere dispenser 2 is filled in use with a plurality of reagent beads or microspheres. 2000 reagent beads or microspheres each having a diameter of 1.75 mm may be loaded into a single reagent bead or microsphere dispenser 2. Other arrangements are contemplated wherein the capacity of the reagent bead or microsphere dispenser 2 may be greater or smaller.

According to another arrangement the reagent bead or microsphere dispensers 2 may be arranged to handle reagent beads or microspheres having a diameter other than 1.75 mm. Arrangements are also contemplated wherein reagent beads or microspheres in a first reagent bead or microsphere dispenser 2 may have a first diameter and wherein reagent beads or microspheres in a second different reagent bead or microsphere dispenser 2 may have a second different diameter. Other arrangements are also contemplated wherein the reagent beads or microspheres loaded into a particular reagent bead or microsphere dispenser 2 may have a plurality or mixture of different diameters.

At least some of the reagent bead or microsphere dispensers 2 comprise a hook 8 which depends from the funnel-shaped dispensing portion 6 and which is arranged to connect or lock with the retaining pin 4 of a docking portion on the carousel 1. An upper portion of the tubular body 5 is arranged to be secured to the docking portion by the clip 3 of the docking portion. The upper clip 3 of at least some of the docking portions may take a different form to that shown in Fig. 1. Other arrangements are contemplated wherein different ways of securing reagent bead or microsphere dispensers 2 to docking portions of the carousel 1 may be used.

Each reagent bead or microsphere dispenser 2 comprises a central auger, helical screw or screw thread mechanism 9 which when rotated translates reagent beads or microspheres from within the tubular body 5 towards the dispensing portion 6. The base of the tubular body 5 which holds reagent beads or microspheres in use comprises an annular disk or base section having a central aperture. The auger, helical screw or screw thread mechanism 9 passes through the central aperture in the base of the tubular body 5. The dispensing portion 6 comprises a tubular bore through which the auger, helical screw or screw thread mechanism 9 passes. The diameter of the tubular bore within the dispensing portion 6 and the pitch of the auger, helical screw or screw thread mechanism 9 are arranged such that reagent beads or microspheres within the dispensing portion 6 are advanced towards the nozzle of the dispensing portion 6 and may be dispensed one at a time from the dispensing portion 6.

A shaft or upper end of the auger, helical screw or screw thread mechanism 9 is connected to a first cog or other first drive mechanism 10. With reference to Fig. 1, the first cog or first drive mechanism 10 at the upper end of the auger, helical screw or screw thread mechanism 9 is arranged to be driven by a corresponding second drive cog 11 or a second drive mechanism which is arranged on an arm 12 of the carousel 1. Teeth on the first cog 10 of the reagent bead or microsphere dispenser 2 engage and interlock with corresponding teeth on the second drive cog 11 of the arm 12 of the carousel 1 so that rotation of the second drive cog 11 on the arm 12 of the carousel 1 causes rotation of the first cog 10 and hence rotation of the auger, helical screw or screw thread mechanism 9 which is connected to the first cog 10.

According to an arrangement each reagent bead or microsphere dispenser 2 is filled with a plurality of reagent beads or microspheres. The reagent beads or microspheres comprise a polystyrene, plastic or polymer core which may be coated with a ferrous or magnetic coating or which have a ferrous or magnetic property. The reagent beads or microspheres may be coated with a reagent (e.g. an antibody or antigen) which is used to analyse samples. The reagent may be used to analyse samples by polymerase chain reactions (PCR) or as part of an immunoassay procedure. Alternatively, the reagent may comprise a DNA or RNA sequence which is used as a hybridization probe to detect the presence of complementary DNA or RNA sequences in a sample. The reagent beads or microspheres may also be coated with an anti-static coating or may have an anti-static property.

One or more sensors may be arranged on the carousel 1 below or close to the dispensing portion 6 or a reagent bead or microsphere dispenser 2. The one or more sensors monitor whether or not one or more reagent beads or microspheres have been dispensed from the dispensing portion 6 into a reagent bead or microsphere receiving chamber of a sample plate 13. The pitch of the auger, helical screw or screw thread mechanism 9 and the speed of rotation of the auger, helical screw or screw thread mechanism 9 is such that individual reagent beads or microspheres can be dispensed from the dispensing portion 6 of a reagent bead or microsphere dispenser 2 in less than 0.5 seconds.

As is shown in Fig. 1, a sample plate 13 is mounted on a translation stage below the arm 12 of the carousel 1. The sample plate 13 comprises a plurality of sample wells. According to an arrangement not in accordance with the present invention each sample well comprises a central fluid receiving area and a plurality of reagent bead or microsphere receiving chambers disposed around the central fluid receiving area. Reagent beads or microspheres from a reagent bead or microsphere dispenser 2 are dispensed into desired reagent bead or microsphere receiving chambers in the sample plate 13. The sample plate 13 is translated by the translation stage so that a desired reagent bead or microsphere receiving chamber is located in close proximity to the nozzle of the dispensing portion 6 of the reagent bead or microsphere dispenser 2. A reagent bead or microsphere is then dispensed into a reagent bead or microsphere receiving chamber and the sample plate 13 is moved by the translation stage so that a different reagent bead or microsphere receiving chamber is disposed in close proximity to the nozzle of the reagent bead or microsphere dispenser 2. The process of dispensing a reagent bead or microsphere and translating the sample plate 13 is then repeated. Once all desired reagent beads or microspheres from a particular reagent bead or microsphere dispenser 2 have been dispensed into appropriate reagent bead or microsphere receiving chambers of the sample plate 13, the carousel 1 is then rotated in order to bring a second desired reagent bead or microsphere dispenser 2 into engagement with the second drive cog 11 disposed on the arm 12 of the carousel 1. Reagent beads or microspheres from the second reagent bead or microsphere dispenser 2 are then dispensed into desired reagent bead or microsphere receiving chambers of the sample plate 13. This process is repeated so that reagent beads or microspheres from further reagent bead or microsphere dispensers 2 attached to the carousel 1 are dispensed into further reagent bead or microsphere receiving chambers in the sample plate 13. Arrangements are also contemplated wherein some of the reagent bead or microsphere dispensers 2 attached to the carousel 1 may be changed or refreshed during the process of dispensing reagent beads or microspheres into the sample plate 13.

Reagent beads or microspheres may be dispensed from reagent bead or microsphere dispensers 2 into the reagent bead or microsphere receiving chambers of the sample plate 13 in any desired manner. For example, in one sample well the same species or type of reagent bead or microsphere may be dispensed into all the reagent bead or microsphere receiving chambers. In another sample well, pairs of the same species of type of reagent bead or microsphere may be dispensed into adjacent reagent bead or microsphere receiving chambers. A single reagent bead or microsphere is dispensed into each reagent bead or microsphere receiving chamber and different types of reagent beads or microspheres are dispensed into each of the reagent bead or microsphere receiving chambers of a particular sample well. However, according to other arrangements some of the reagent bead or microsphere receiving chambers may be left empty. It is also contemplated that according to other arrangements some reagent bead or microsphere receiving chambers may receive more than one reagent bead or microsphere particularly if the reagent beads or microspheres concerned have a relatively small diameter relative to other reagent beads or microspheres which may be dispensed into other reagent bead or microsphere dispensing chambers.

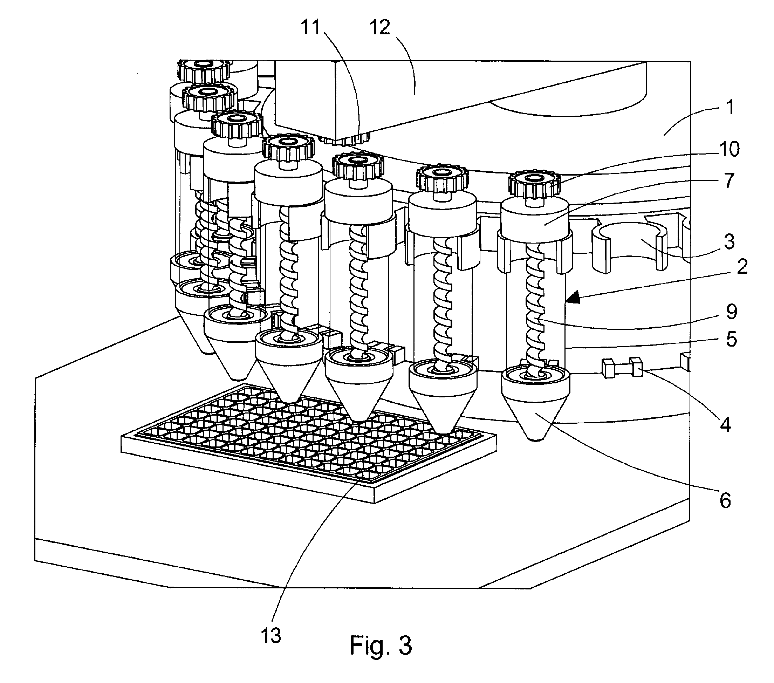

Fig. 3 shows in greater detail a plurality of reagent bead or microsphere dispensers 2 secured to docking portions on the carousel 1 by a clip 3 and a retaining pin 4. The retaining pin 4 engages with a hook 8 provided on the dispensing portion 6 of the reagent bead or microsphere dispenser 2. The retaining pin 4, hook 8 and clip 3 prevent the body of a reagent bead or microsphere dispenser 2 from rotating during use. The auger, helical screw or screw thread mechanism 9 within each reagent bead or microsphere dispenser 2 is rotated or driven by bringing the teeth of a first cog 10 attached to the spindle or shaft of the auger, helical screw or rotating mechanism 9 into intermeshing or interlocking engagement with a second drive cog or second drive mechanism 11 which depends from the arm 12 of the carousel 1. The second drive cog or second drive mechanism 11 is driven or rotated by an electric motor.

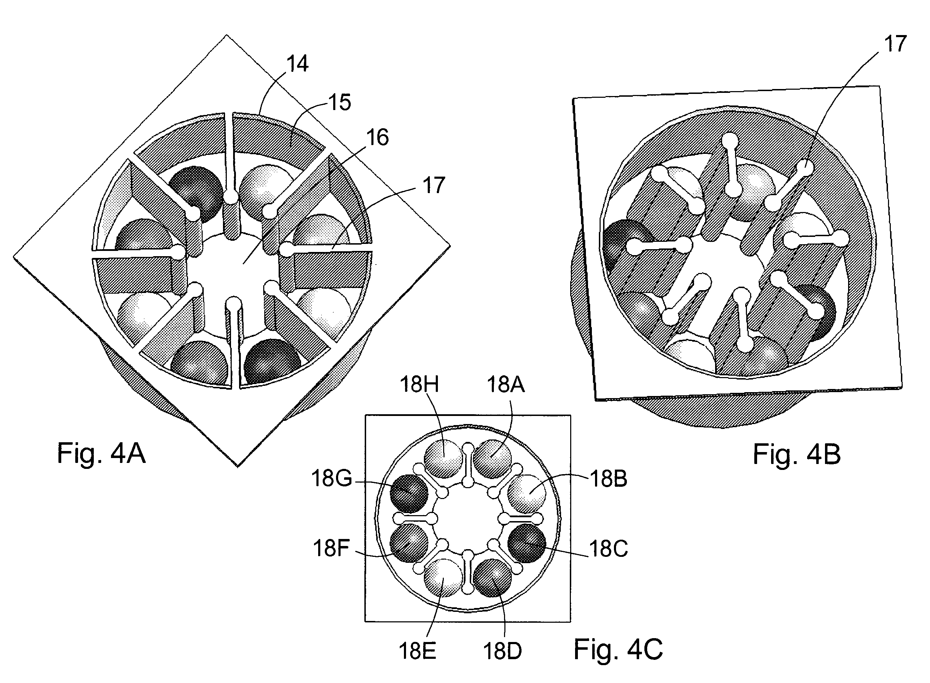

Fig. 4A shows an individual sample well 14 of a sample plate 11 not in accordance with the present invention. According to the particular arrangement illustrated in Fig. 4A, the sample well 14 may comprise eight reagent bead or microsphere receiving chambers 15 disposed around a central fluid receiving area 16. Other arrangements are contemplated wherein a different number of reagent bead or microsphere receiving chambers or regions 15 are provided. Each reagent bead or microsphere receiving chamber 15 is defined by at least two radial wall members 17 together with the outer or inner wall of the sample well 14. The radial wall members 17 depend from the wall of the sample well 14 and extend towards the centre of the sample well 14. However, the wall members 17 do not extend all the way to the centre of the sample well 14 so that a central circular fluid receiving area 16 is provided. At least some or all of the radial wall members 17 which terminate adjacent the central fluid receiving area 16 may comprise an enlarged portion which is designed to assist in the retention of reagent beads or microspheres within their individual reagent bead or microsphere receiving chambers 15 and to prevent the reagent beads or microspheres from passing into the fluid receiving area 16. Other arrangements are contemplated wherein the height of at least some or substantially all of the radial wall members 17 merely reduces in the region of the central fluid receiving area 16.

In the arrangement shown in Fig. 4A the radial wall members 17 depend from the outer or inner wall of the sample well 14. However, other arrangements not in accordance with the present invention are contemplated such as the arrangement illustrated in Fig. 4B wherein the radial wall members 17 do not depend from the wall of the sample well 14. Instead, the radial wall members 17 are spaced apart from the outer or inner wall of the sample well 14. At least some or all of the radial wall members 17 which terminate short of the outer or inner wall of the sample well 14 may comprise an enlarged portion which is designed to assist in the retention of reagent beads or microspheres within their individual reagent bead or microsphere receiving chambers 15. Other arrangements are contemplated wherein the height of at least some or substantially all of the radial wall members 17 merely reduces towards the outer or inner wall of the sample well 14.

Fig. 4C shows an arrangement not in accordance with the present invention herein eight different types of reagent beads or microspheres are shown dispensed into separate reagent bead or microsphere receiving chambers 15 of a sample well. In the particular arrangement shown in Fig. 4C, first reagent bead or microsphere 18A is coated with a first reagent, second reagent bead or microsphere 18B is coated with a second different reagent, third reagent bead or microsphere 18C is coated with a third different reagent, fourth reagent bead or microsphere 18D is coated with a fourth different reagent, fifth reagent bead or microsphere 18E is coated with a fifth different reagent, sixth reagent bead or microsphere 18F is coated with a sixth different reagent, seventh reagent bead or microsphere 18G is coated with a seventh different reagent and eighth reagent bead or microsphere 18H is coated with an eighth different reagent. Therefore, according to this arrangement eight individually chosen and distinct immunoassay procedures can be performed substantially simultaneously on a single fluid sample so that multiplexed testing can be performed.

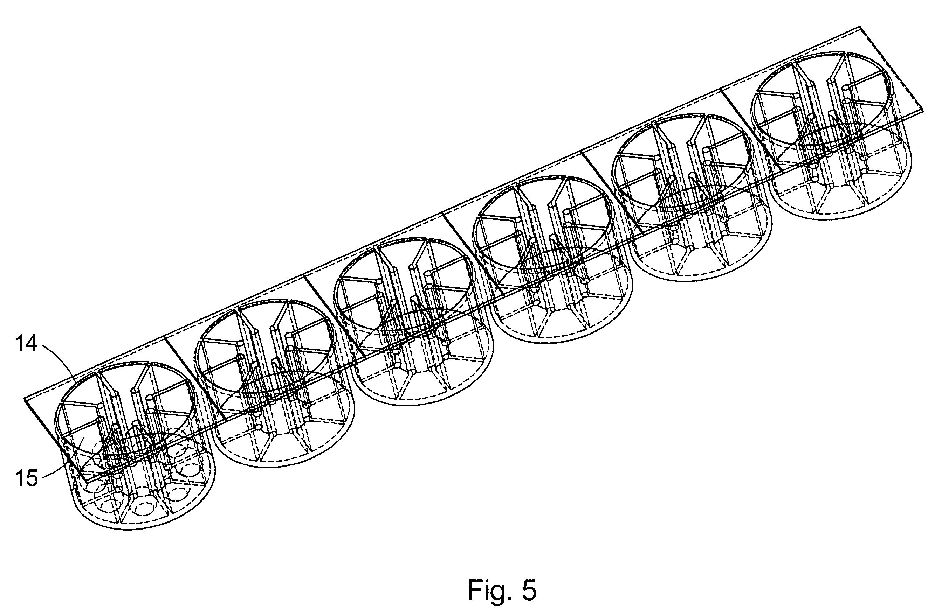

Fig. 5 shows a further arrangement not in accordance with the present invention wherein a sample plate comprising a strip of six sample wells 14 is provided. Each sample well 14 comprises eight reagent bead or microsphere receiving chambers 15.

Although the above sample well 14 comprises a plurality of radial or straight walls 17, other arrangements not in accordance with the present invention are contemplated wherein the walls separating adjacent reagent bead or microsphere receiving chambers 15 may be curved. According to a yet further arrangement the reagent bead or microsphere receiving chambers 15 may have a honeycombed structure formed of a plurality of polygonal (e.g. hexagonal) chambers and/or may comprise a plurality of circular reagent bead or microsphere receiving chambers 15.

According to the above arrangement a fluid to be tested is dispensed into the central fluid receiving area 16 of a sample well 14. The fluid may, for example, comprise a sample of blood, serum, saliva or urine taken from a patient. The fluid which is dispensed into the central fluid receiving area 16 of the sample well 14 flows into each of the adjoining reagent bead or microsphere receiving chambers 15 by flowing between the gap between two radial wall members 17 which help to define a reagent bead or microsphere receiving chamber 15. According to the arrangement the fluid which is dispensed does not flow over the top of the radial wall members 17.

At least some of the reagent beads or microspheres which are dispensed into the reagent bead or microsphere receiving chambers 15 of a sample well 14 may have a ferrous or magnetic layer or coating and/or have a ferrous or magnetic property. A magnetic or electro-static device may be used to attract reagent beads or microspheres as they are being dispensed from a reagent bead or microsphere dispenser 2 in order to guide the reagent beads or microspheres 2 being dispensed into an appropriate reagent bead or microsphere receiving chamber 15 of a sample well 14. Once reagent beads or microspheres have been dispensed into a reagent bead or microsphere receiving chamber 15 the magnetic or electro-static device may then be used to attract, retain or otherwise hold the reagent beads or microspheres in their reagent bead or microsphere receiving chambers 15 for a period of time.

Other arrangements are contemplated wherein a mechanical device or an electrical device may be used to funnel or direct reagent beads or microspheres into appropriate reagent bead or microsphere receiving chambers 15 and/or to retain or otherwise hold reagent beads or microspheres which have been dispensed into reagent bead or microsphere receiving chambers 15 in their chamber 15 for a period of time.

According to a further arrangement not in accordance with the present invention a magnetic, electro-static, mechanical or electrical device may be used to vibrate or agitate reagent bead or microspheres which have been dispensed into appropriate reagent bead or microsphere receiving chambers 15. According to an arrangement not in accordance with the present invention reagent beads or microspheres located in reagent bead or microsphere receiving chambers 15 may be vibrated or agitated once a fluid sample has been dispensed into the central fluid receiving area 16 and once the fluid sample has dispersed into each of the various reagent bead or microsphere receiving chambers 15. This process helps to ensure that the various reagent beads or microspheres are completely wetted or otherwise coated by the dispensed fluid sample. 10-200 ml of fluid sample may be dispensed into each of the central fluid receiving areas 16 of the sample wells 14 comprising a sample plate 13.

In addition to or as an alternative to a sensor disposed on the carousel 1 or otherwise arranged in close proximity to the dispensing portion 6 of a reagent bead or microsphere dispenser 2, a visual detection system may be used to determine whether or not one or more reagent beads or microspheres have been dispensed or are otherwise correctly located in appropriate reagent bead or microsphere receiving chambers 15 of a sample plate 13. According to an arrangement the reagent beads or microspheres may be coloured and may contrast with the colour of the sample plate 13 which according to arrangement is substantially clear. The sample plate 13 may comprise one or more luminescence or fluorescence markers and a luminescence or fluorescence detecting device may be used to determine whether or not reagent beads or microspheres have been correctly dispensed into appropriate reagent bead or microsphere receiving chambers 15 of a sample well 14. A determination may be made, for example, by determining whether or not a reagent bead or microsphere obscures or obstructs the luminescence or fluorescence markers on the sample plate 13 from being observed or otherwise detected. Other arrangements are contemplated wherein a magnetic, electrical, capacitive or mechanical sensor may be used to determine the presence or absence of reagent beads or microspheres in reagent bead or microsphere receiving chambers 15 of a sample plate 14.

A control system may be used to determine the number and/or location and/or type of reagent beads or microspheres which have been dispensed into reagent bead or microsphere receiving chambers 15. The control system may also determine into which reagent bead or microsphere receiving chambers 15 further reagent beads or microspheres should be dispensed. Once sample fluid has been dispensed into the central fluid receiving areas of sample wells 14, the control system may check that an appropriate amount of sample fluid has been dispensed and that all the reagent beads or microspheres are at least partially or are fully immersed by the sample fluid.

The amount of sample fluid to be dispensed into the central fluid receiving area of a sample well 14 may depend upon the number of reagent bead or microsphere receiving chambers 15 formed within the sample well 14, the diameter of the reagent beads or microspheres which are dispensed into the reagent bead or microsphere receiving chambers 15 and the number of reagent beads or microspheres which are dispensed into any given sample well 14. The control system may be used to vary the amount of sample fluid dispensed into sample wells 14 so that reagent beads or microspheres are immersed in sample fluid to a substantially constant depth irrespective of the number of reagent beads or microspheres present in a sample well 14, the number of reagent bead or microsphere receiving chambers 15 and the diameter of the reagent beads or microspheres which are dispensed.

Different formats of sample plates 13 may be provided. For example, as shown in Figs. 1 and 3 the sample plate 13 may comprise a two dimensional array of sample wells 14. For example, the sample plate 13 may comprise a 4x4, 4x6, 4x8, 4x10, 4x12, 6x6, 6x8, 6x10, 6x12, 8x8, 8x10, 8x12, 10x10, 10x12 or 12x12 array of sample wells 14. The sample plate 13 may alternatively comprise a single dimensional strip of sample wells 14. For example, the sample plate 13 may comprise a 4x1, 6x1, 8x1, 10x1 or 12x1 strip of sample wells 14. Yet further arrangements are contemplated wherein the sample wells 14 may be provided in a format other than in an array or strip.

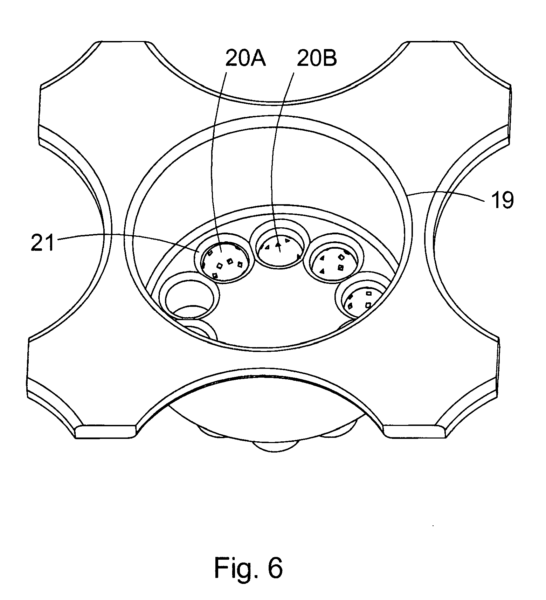

A preferred embodiment of the present invention will now be described with reference to Fig. 6. According to the preferred embodiment a sample plate is provided which preferably comprises a plurality of sample wells 19 (although according to another less preferred embodiment a sample plate may be provided which comprises only a single sample well 19). According to a preferred embodiment the sample plate may comprise a 9x6 array of sample wells 19. A single sample well 19 is shown in Fig. 6. Embodiments are also contemplated wherein the sample plate may comprise a strip of sample wells 19 e.g. the sample plate may comprise, for example, a 1 x9 or 1x6 array or strip of sample wells 19.

Each sample well 19 preferably comprises a plurality of pockets, recesses or bores 21 which are preferably provided in the base of the sample well 19. In the particular embodiment shown in Fig. 6 the sample well 19 comprises ten pockets, recesses or bores 21 which are formed or otherwise provided in the base of a sample well 19. Other embodiments are contemplated wherein a different number of pockets, recesses or bores 21 may be provided in the base of the sample well 19. For example, according to alternative embodiments at least some or all of the sample wells 19 provided in a sample plate may comprise 1, 2, 3, 4, 5, 6, 7, 8, 9, 11, 12, 13, 14, 15, 16, 17, 18, 19, 20 or > 20 pockets, recesses or bores 21.

The pockets, recesses or bores 21 are preferably provided around the edge or perimeter of the sample well 19 and the centre or central region of the base of the sample well 19 is preferably substantially flat and free from pockets, recesses or bores 21. According to the arrangement described above with reference to Figs. 1-5 and not in accordance with the present invention the sample plate comprised a plurality of radial wall members in order to retain reagent beads or microspheres in their respective reagent bead or microsphere receiving chamber. However, according to the preferred embodiment reagent beads or microspheres are preferably secured within the pockets, recesses or bores 21 of the sample plate 19 and hence radial wall members are not required and hence are preferably not provided.

Other embodiments are also contemplated wherein a hybrid between a conventional microplate and a sample plate according to the above arrangement or the preferred embodiment may be provided. For example, according to an embodiment a sample plate may be provided which comprises one or more conventional sample wells and one or more sample wells having a plurality of recesses, pockets or bores for receiving a reagent bead or microsphere.

Referring to the preferred embodiment as illustrated in Fig. 6, at least some or all of the pockets, recesses or bores 21 which are provided in the base of a sample well 19 preferably comprise a bore which is preferably tapered along at least a portion or substantially the whole of its length. The pockets, recesses or bores 21 may, for example, be arranged to have a 6° taper. According to an embodiment the top (or reagent bead or microsphere receiving portion) of the tapered bore may have a diameter of 1.82 mm. The base of the sample well 19 surrounding the bore may be arranged to have a countersunk portion in order to facilitate the insertion of a reagent bead or microsphere 20A;20B into the pocket, recess or bore 21. According to an embodiment the outer diameter of the countersunk portion may be 2.25 mm.

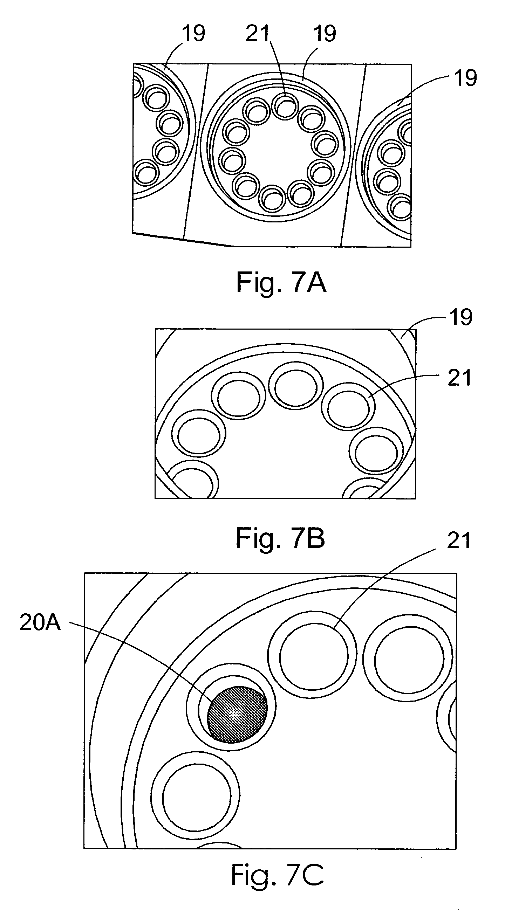

Fig. 7A shows a plan view of a sample well 19 and portions of two adjacent sample wells 19 which are provided in a sample plate according to the preferred embodiment of the present invention. The sample wells shown in Fig. 7A form part of an array of sample wells 19 which are provided in the sample plate. Each of the sample wells 19 comprise ten pockets, recesses or bores 21 which are disposed in the bottom or base portion of the sample well 19. In use reagent beads or microspheres are preferably inserted into each of the pockets, recesses or bores 21 of a sample well 19 and the reagent beads or microspheres are preferably secured in the pockets, recesses or bores 21 by virtue of the diameter of the bore tapering and becoming restricted.

Fig. 7B shows in greater detail the bottom of a sample well 19 and shows a plurality of pockets, recesses or bores 21 provided in the bottom portion of the sample well 19 each of which are arranged and adapted to receive a reagent bead or microsphere. Each of the pockets, recesses or bores 21 provided in the base of the sample well 19 preferably also comprises a countersunk portion or region at the entrance to each tapered bore. According to the preferred embodiment a single reagent bead or microsphere is dispensed and inserted into each pocket, recess or bore 21.

Fig. 7C shows in further detail a reagent bead or microsphere 20A disposed and securely located in a pocket, recess or bore 21 provided in the base of a sample well 19 according to the preferred embodiment of the present invention. The reagent bead or microsphere 20A is secured within the pocket, recess or bore 21 and the upper surface of the reagent bead or microsphere 20A when secured or located within the pocket, recess or bore 21 is positioned or located approximately 0.3 mm below the surface of the well bottom. Therefore, according to the preferred embodiment reagent beads or microspheres 20A located and secured in the pockets, recesses or bores 21 provided in the bottom of a sample well 19 preferably do not project above the entrance to or surface of the pocket, recess or bore 21 and hence preferably do not project above the bottom surface of the sample well 19. However, less preferred embodiments are contemplated wherein one or more reagent beads or microspheres located in one or more pockets, recesses or bores 21 provided in the base of the sample well 19 may be located in relatively shallow pockets, recesses or bores 21 or may be located in one or more pockets, recesses or bores 21 which have a taper such that when the reagent bead or microsphere 20A is securely positioned within the pocket, recess or bore 21 then the reagent bead or microsphere projects slightly above the entrance into or surface of the pocket, recess or bore 21 and hence projects above the bottom surface of the sample well 19.

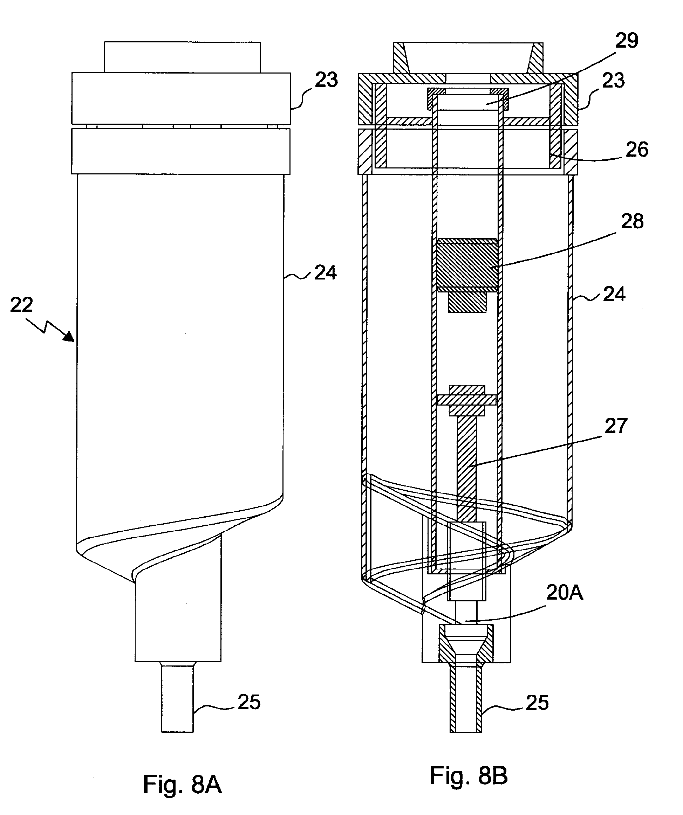

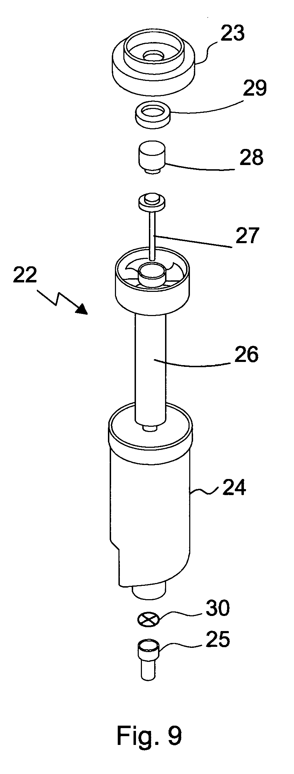

Reagent beads or microspheres are preferably dispensed into pockets, recesses or bores 21 provided in the bottom of a sample well 19 of a sample plate by means of a reagent bead or microsphere dispenser 22 as will now be described with reference to Figs. 8A, 8B and 9. A preferred reagent bead or microsphere dispenser 22 is shown in Fig. 8A and preferably comprises an upper cap 23, a syringe body 24 and a barrel 25 which projects from a lower region of the syringe body 24.

Fig. 8B shows a cutaway view of the reagent bead or microsphere dispenser 22 and shows that the reagent bead or microsphere dispenser further comprises a plunger guide 26 which is preferably positioned within the body of the syringe body 24. The plunger guide 26 preferably comprises a screw thread on the outer surface of an upper portion of the plunger guide 26. The inner surface of an upper portion of the syringe body 24 preferably comprises a complementary screw thread which engages with the screw thread provided on the outer surface of the upper portion of the plunger guide 26 so that in use the plunger guide 26 is secured or screwed firmly to the syringe body 24. The inner surface of the cap 23 also preferably comprises a screw thread and the cap 23 also preferably screws onto the upper portion of the plunger guide 26.

A plunger 27 is preferably located within the plunger guide 26 and the plunger 27 may be depressed by actuating an actuator or plunger boss 28 which is located above the plunger 27 in the bore defined by the plunger guide 26. An actuator spring (not shown) is provided between the actuator or plunger boss 28 so that when the actuator or plunger boss 28 is depressed, force is transmitted to the plunger 27 via the actuator spring causing the plunger 27 to become depressed. A return spring (not shown) is preferably provided between the bottom portion of the plunger guide 26 and the plunger 27 so that when the actuator or plunger boss 28 is no longer depressed, both the plunger 27 and the actuator or plunger boss 28 are preferably returned to an upper position.

Fig. 9 shows an exploded view of the reagent bead or microsphere dispenser 22 as shown and described above with reference to Figs. 8A and 8B. Fig. 9 also shows that a silicone member 30 is preferably provided within the upper portion of the barrel 25. In use, reagent beads or microspheres within the syringe body 24 are preferably funnelled or channelled by a helical path formed in the bottom section of the syringe body 24 so that at the bottom of the syringe body 24 reagent beads or microspheres become arranged in single file or in series. The single file or series of reagent beads or microspheres leads into a chamber which is preferably arranged immediately above the barrel 25 and below the plunger guide 26. The chamber is shaped and arranged so as to accommodate a single reagent bead or microsphere which is positioned in a bore below the plunger 27 and above the barrel 25. When the plunger 27 is depressed, the plunger 27 preferably pushes a single reagent bead or microsphere 20A located in the chamber in a downwards direction. The single reagent bead or microsphere 20A is preferably forced by the plunger 27 through the silicone member 30. The plunger 27 preferably continues to push or urge the reagent bead or microsphere 20A through the barrel 25 and into a pocket, recess or bore 21 of a sample well 19 which is preferably positioned immediately below the barrel 25 of the reagent bead or microsphere dispenser 22. The silicone member 30 preferably prevents the accidental release of reagent beads or microspheres from the chamber of the reagent bead or microsphere dispenser 22 into the barrel 25 of the syringe body 24.

The bottom portion of the syringe body 24 preferably has a helical shape and acts to guide or channel reagent beads or microspheres towards the chamber disposed in a lower portion of the syringe body 24. The chamber is preferably arranged so that only a single reagent bead or microsphere sits above the silicone member 30 at any instance in time. The chamber is formed in the bore through which the plunger 27 travels and depression of the plunger 27 preferably causes a reagent bead or microsphere located in the chamber to be urged through the silicone member 30 and into the barrel 25.

A vibration mechanism may optionally be provided and may be arranged to act on the outside of the syringe body 24 so as to ensure that reagent beads or microspheres move down through syringe body 24 to the bottom portion of the syringe body 24 and line up in single file or in series ready to enter the chamber.

Reagent beads or microspheres may be pre-packed or pre-loaded into the syringe body 24 by, for example, a kit manufacturer or other supplier. Alternatively, an end-user may load the syringe body 24 with reagent beads or microspheres.

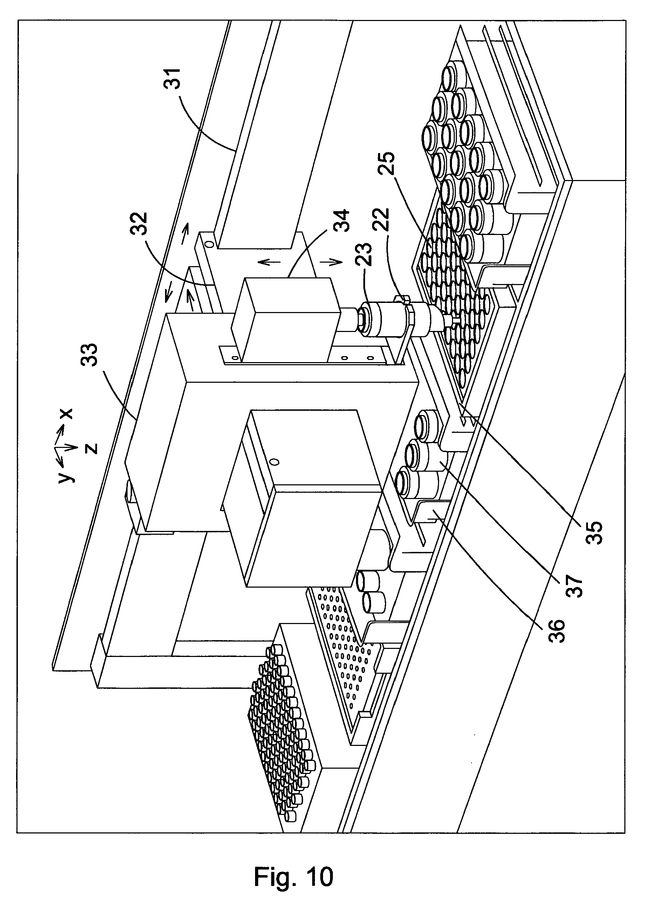

A microarrayer or automated apparatus will now be described with reference to Fig. 10. As shown in Fig. 10, a plurality of syringe bodies 37 may be loaded onto a tray or pack 36 which is then preferably automatically loaded into the microarrayer or automated apparatus. The tray or pack 36 comprising a plurality of syringe bodies 37 may be moved by a three-axis translation mechanism or robotic arm to a reagent bead or microsphere dispensing work area of the microarrayer or automated apparatus.

The microarrayer or automated apparatus preferably comprises a three-axis translation mechanism which preferably comprises a first translation stage comprising a guide rail 31 along which a first arm 32 may be translated in a first (x) horizontal direction. A second translation stage is preferably provided and comprises a mounting block 33 which preferably encompasses or surrounds the first arm 32. The mounting block 33 may be translated in a second (y) horizontal direction (which is preferably orthogonal to the first (x) horizontal direction) and may be moved backwards and forwards along the first arm 32. A third translation stage is preferably provided and preferably comprises a body or syringe drive mechanism 34 which preferably houses a linear actuator (not shown). The body or syringe drive mechanism 34 is preferably slidably mounted on the mounting block 33 and may be raised and lowered in a vertical (z) direction.

The three-axis translation mechanism preferably further comprises a retractable arm 35 which preferably extends from the mounting block 33. The three-axis translation mechanism is preferably programmed to select and pick up a reagent bead or microsphere dispenser 22,37 from the tray or pack 36 comprising a plurality of reagent bead or microsphere dispensers 22,37. The body or syringe drive mechanism 34 comprises a tapered spigot which is resiliently mounted within a tubular housing. The spigot is arranged to engage with a tapered portion provided on the syringe cap 23 of the reagent bead or microsphere dispenser 22,37. When a reagent bead or microsphere dispenser 22,37 is positioned in the tray or pack 36 the spigot may be lowered onto the syringe cap 23 of a reagent bead or microsphere dispenser 22,37 thereby securing the reagent bead or microsphere dispenser 22,37 to the body or syringe drive mechanism 34 in a detachable manner. The body or syringe drive mechanism 34 and attached reagent bead or microsphere dispenser 22,37 may then be raised to a height such that the retractable arm 35 (which is initially retracted within the body of the mounting block 33) can then be extended. The reagent bead or microsphere dispenser 22,37 is then lowered by the body or syringe drive mechanism 34 so that the upper portion of the syringe body 24 is secured by the retractable arm 35. The retractable arm 35 preferably has an aperture having an internal diameter which is preferably smaller than the outermost diameter of a rim of the upper portion of the syringe body 24.

Each reagent bead or microsphere dispenser 22,37 preferably comprises a plurality of identical reagent beads or microspheres. According to an embodiment up to 15 separate reagent bead or microsphere dispensers 22,37 may be loaded or provided in a single tray or pack 36 and each of the reagent bead or microsphere dispensers 22,37 may have a capacity of up to approximately 2000 reagent beads or microspheres.

The syringe drive mechanism 34 is arranged to pick a reagent bead or microsphere dispenser 22,37 out of the tray or pack 36 and will position and lower the barrel 25 of the reagent bead or microsphere dispenser 22,37 so that it is immediately above a desired reagent bead or microsphere pocket or recess 21 provided in a sample well 19 of a sample plate. The syringe drive mechanism 34 is then preferably actuated so that the actuator or plunger boss 28 of the reagent bead or microsphere dispenser 22,37 is depressed which in turn causes the plunger 27 to push a reagent bead or microsphere 20A from the chamber through the silicone member 30, through the barrel 25 and into the desired reagent bead or microsphere pocket or recess 21 of the sample well 19. The syringe drive mechanism 34 is preferably arranged to depress the actuator boss 28 and plunger 27 with a desired amount of force as opposed to moving the actuator or plunger boss 28 and plunger 27 to a certain vertical position. As a result, reagent beads or microspheres 20A are preferably pressed in tightly and consistently into the reagent bead or microsphere pockets or recesses 21 of a sample well 19 with a constant amount of force.

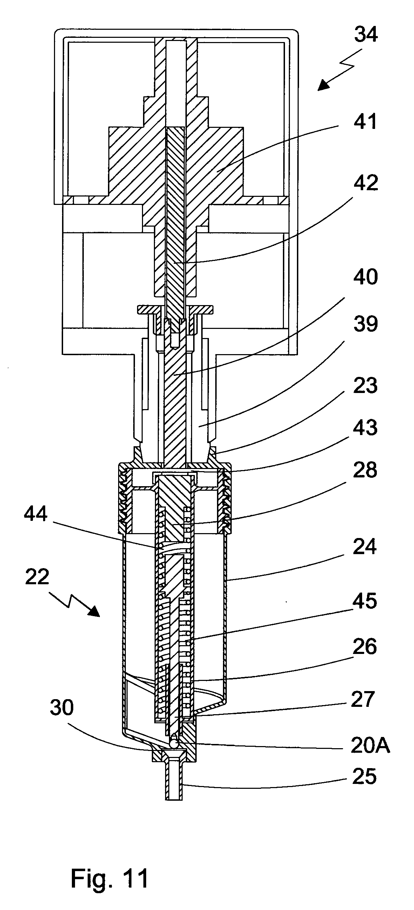

Fig. 11 shows in greater detail a reagent bead or microsphere dispenser pick-up device or syringe drive mechanism 34 during the process of picking up a reagent bead or microsphere dispenser 22. The reagent bead or microsphere dispenser pick-up device or syringe drive mechanism 34 comprises a spigot 39 having a tapered lower end which is arranged to engage with a tapered recess provided in the upper portion of the syringe cap 23 of the reagent bead or microsphere dispenser 22. The spigot 39 comprises a central bore through which a plunger push rod 40 is mounted. The plunger push rod 40 is arranged to be driven upwards or downwards by a linear actuator 41 which drives a linear actuator lead screw 42 which in turn raises or lowers the plunger push rod 40.

As shown in Fig. 11, in order to pick up a reagent bead or microsphere dispenser 22 the reagent bead or microsphere dispenser pick-up device or syringe drive mechanism 34 is lowered onto the reagent bead or microsphere dispenser 22 so that the spigot 39 of the reagent bead or microsphere pick-up device or syringe drive mechanism 34 engages with the syringe cap 23 of the reagent bead or microsphere dispenser 22. As the reagent bead or microsphere dispenser pick-up device or syringe drive mechanism 34 is driven downwards onto the reagent bead or microsphere dispenser 22, the spigot 39 becomes compressed and moves upwards until it is prevented from moving any further upwards. The spigot 39 is preferably driven further downwards whilst in a compressed state so that the interlocking tapers of the spigot 39 and syringe cap 23 preferably engage causing the reagent bead or microsphere dispenser 22 to become attached to the reagent bead or microsphere pick-up device or syringe drive mechanism 34.

The reagent bead or microsphere dispenser 22 as shown in Fig. 11 is substantially similar to that shown in Figs. 8A, 8B and 9 except that the spacer 29 shown in Figs. 8B and 9 is replaced with a retaining cap 43 in the embodiment shown in Fig. 11. Fig. 11 also shows the location of an actuating spring 44 which is provided between the actuator or plunger boss 28 and the plunger 27 and which transmits force applied to the actuator or plunger boss 28 to the plunger 27. A return spring 45 is also shown and is provided between the plunger 27 and the base of the plunger guide 26 and causes the plunger 27 (and hence also the actuator or plunger boss 28) to return to an upper position when the actuator or plunger boss 28 is no longer depressed or actuated.

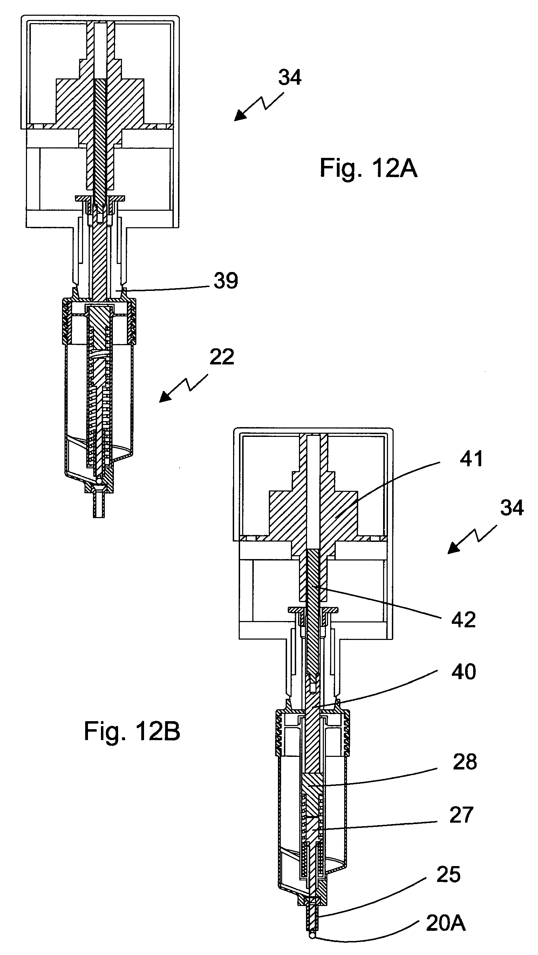

Fig. 12A shows the reagent bead or microsphere dispenser pick-up device or syringe drive mechanism 34 which has picked up a reagent bead or microsphere dispenser 22 and which is in the process of transporting the reagent bead or microsphere dispenser 22 to a desired location. Once the reagent bead or microsphere dispenser pick-up device or syringe drive mechanism 34 has engaged with the reagent bead or microsphere dispenser 22, the reagent bead or microsphere dispenser pick-up device or syringe drive mechanism 34 is raised so that the spigot 39 is no longer compressed. The spigot 39 returns to a downward position and the reagent bead or microsphere dispenser 22 including syringe body 24 is locked on to the spigot 39 by the tapers on the spigot 39 and syringe cap 23.

Fig. 12B shows a reagent bead or microsphere dispenser 22 in the process of dispensing a reagent bead or microsphere 20A from the reagent bead or microsphere dispenser 22 into a pocket or recess of a sample well (not shown) of a sample plate (not shown). The linear actuator 41 of the reagent bead or microsphere dispenser pick-up device or syringe drive mechanism 34 is preferably actuated and causes the linear actuator lead screw 42 to extend thereby pushing the push rod 40 downwards. The downwards movement of the push rod 40 depresses the actuator or plunger boss 28. The actuator or plunger boss 28 transmits force to the plunger 27 via the actuating spring 44 and preferably does not touch the plunger 27 directly. The plunger 27 preferably forces a reagent bead or microsphere 20A from a chamber within the central bore provided within the syringe body 24. The reagent bead or microsphere 20A is preferably forced through the membrane 30 and down through the barrel 25 and into the recess or pocket of a sample plate (not shown) by the plunger 27.

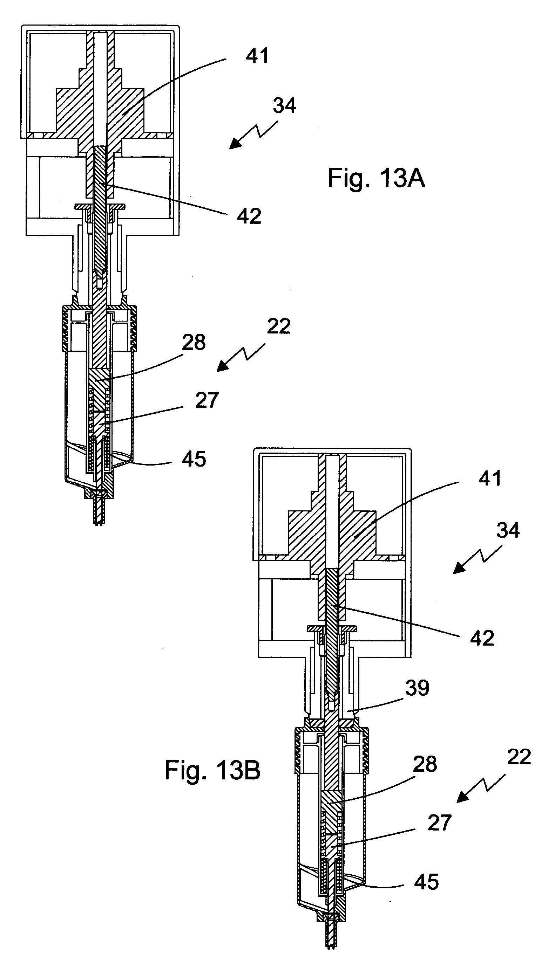

Fig. 13A shows the reagent bead or microsphere pick-up device or syringe drive mechanism 34 in the process of ejecting a reagent bead or microsphere dispenser 22 from the end of the reagent bead or microsphere pick-up device or syringe drive mechanism 34. In this mode of operation the reagent bead or microsphere dispenser 22 is positioned above the tray or pack 36. The linear actuator 41 preferably drives the linear actuator lead screw 42 downwards until the plunger 27 is extended a maximum extent. The spigot 39 is also extended to the maximum extent. The linear actuator 41 then preferably continues to apply force via the actuator or plunger boss 28 to the plunger 27, as shown in Fig. 13B, with the result that the body of the reagent bead or microsphere dispenser 22 is preferably forced off from the end of the tapered spigot 39. The reagent bead or microsphere dispenser 22 then preferably falls back into the reagent bead or microsphere dispenser tray or pack 36.

In order to illustrate aspects of an embodiment of the present invention a test was performed wherein a sample plate comprising nine sample wells 19 was provided. Each sample well 19 comprised ten pockets, recesses or bores 21 which were arranged in a circle around a central portion of the sample well 19. Each of the pockets, recesses or bores 21 were loaded with reagent beads or microspheres which were coated with different concentrations of reagent. The ten beads in the first sample well were coated with a reagent having a concentration of 10 µg/ml and the ten beads in the second sample well were coated with a reagent having a concentration of 8 µg/ml. The ten beads in the third sample well were coated with a reagent having a concentration of 4 µg/ml and the ten beads in the fourth sample well were coated with a reagent having a concentration of 2 µg/ml. The ten beads in the fifth sample well were coated with a reagent having a concentration of 1 µg/ml and the ten beads in the sixth sample well were coated with a reagent having a concentration of 0.5 µg/ml. The ten beads in the seventh sample well were not coated with a reagent i.e. the concentration was 0 µg/ml. The ten beads in the eighth sample well were coated with different concentrations of reagent and comprised concentrations of 10 µg/ml, 8 µg/ml, 4 µg/ml, 2 µg/ml, 1 µg/ml, 0.5 µg/ml, 0 µg/ml, 0 µg/ml, 0 µg/ml and 0 µg/ml. The ten beads in the ninth sample well had the same concentrations as the reagent beads or microspheres in the eighth sample well and were arranged in the same manner as the reagent beads or microspheres in the eighth sample well.

The reagent beads or microspheres were coated with a capture antibody comprising sheep IgG and were transported in a bicarbonate buffer containing 0.02% Kathon (RTM) preservative.

The sample wells 19 of the sample plate were emptied of the preservative in which the reagent beads or microspheres were transported in and 400 µl of a 1/1000 diluted donkey anti-sheep IgG peroxidise conjugate in a Tris Buffered Saline ("TBS") conjugate diluent buffer was added to each sample well 19. The sample plate was then incubated at ambient temperature and was subjected to medium intensity vibrations for a period of 45 minutes. Any unbound conjugate was then aspirated from the sample wells 19 using a single channel wash head of a microarrayer apparatus (DS2 (RTM), available from Dynex Technologies). Once any unbound conjugate had been aspirated from the sample wells 19, 500 µl of 1/20 diluted Tris Buffered Saline wash fluid was then immediately added to each sample well 19. The wash fluid was then aspirated from the sample wells 19 and the process of washing and aspirating wash fluid from the sample wells 19 was repeated twice more. After the third washing step including aspiration of wash fluid had been completed, 300 µl of luminol (a chemiluminescent marker) was then immediately added to each sample well 19. The sample plate was then incubated in the dark at ambient temperature whilst being subjected to medium intensity vibrations for 15 minutes. The sample plate was then transferred immediately to a reading chamber.

A camera was set to an exposure time of 6 minutes and 30 seconds with a gain of 20. Images were taken at 22 minutes and 29 minutes after luminol had been added. The camera exposure time was then changed to 8 minutes and 37 seconds. Further images were taken at 38 minutes, 47 minutes, 56 minutes and 65 minutes after luminol addition. Analysis of the images showed that the greatest observed signal strength was obtained after 15-22 minutes from luminol addition which is consistent with the luminol decay curve.

The following steps may be carried out once reagent beads or microspheres have been dispensed into pockets, recesses or bores of a sample plate. Firstly, sample fluid may be added to one or more sample wells of the sample plate. The sample fluid may comprise one or more analytes such as specific antigens which may react with reagent coated on one or more of the reagent beads or microspheres. The reagent beads or microspheres are preferably coated with a specific capture antibody.

Once the sample fluid has been added to the sample wells, the sample plate is then preferably subjected to an incubation step. After the sample plate has been subjected to an incubation step so that antigen-antibody complexes are formed, the sample plate is then preferably subjected to one or more washing and aspirate steps in order to remove any unbound sample fluid and to remove any wash fluid. An enzyme conjugate is then added which will bind to the antigen part of any antigen-antibody complexes which have been formed but which will not bind to antibodies or to the antibody part of an antigen-antibody complex. The sample plate is then incubated before being subjected to one or more washing and aspirate steps. Once the sample plate has been subjected to one or more washing and aspirate steps luminol (or another visualising agent) is preferably added. The sample plate is then preferably aspirated to remove any excess luminol (or other visualising agent). The luminol (or other visualising agent) upon contacting enzymes attached to the antigen part of an antigen-antibody complex will then breakdown causing a distinctive colour to be produced. In the final stage the sample plate is analysed and an end point determination is preferably made.

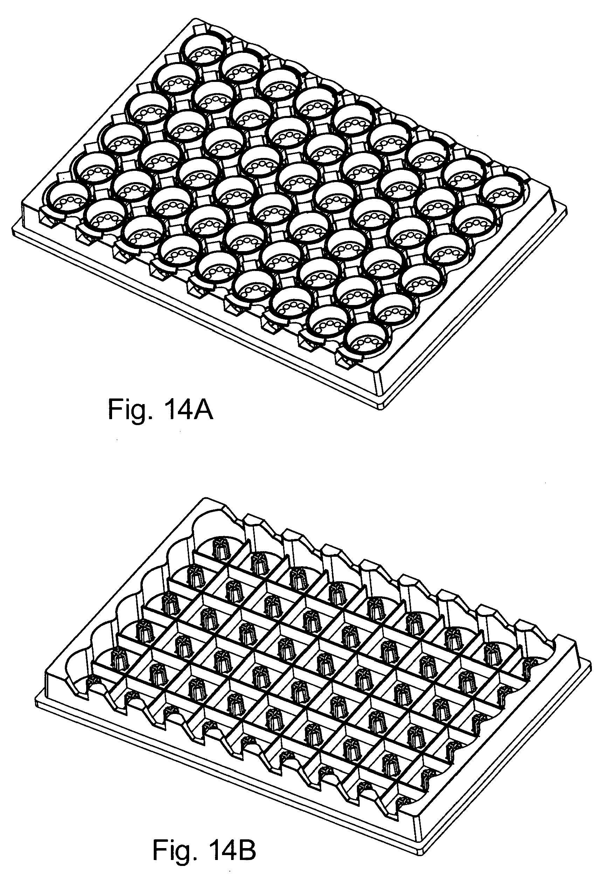

A particularly preferred embodiment of the present invention is shown in Figs. 14A and 14B and will be described in more detail below. Fig. 14A shows nine sample plates loaded into a plate frame. Each of the sample plates shown in Fig. 14A comprises a 6x1 strip of sample wells. The sample plates can be removeably loaded into the plate frame. Each of the nine sample plates or strips comprises six sample wells and each sample well preferably comprises ten tapered bores which, in use, are arranged to receive a reagent bead. The reagent beads are preferably loaded into the tapered bores such that the reagent beads do not protrude above the base portion of the sample well. Fig. 14B shows the plate frame into which the sample plates may be loaded in more detail.

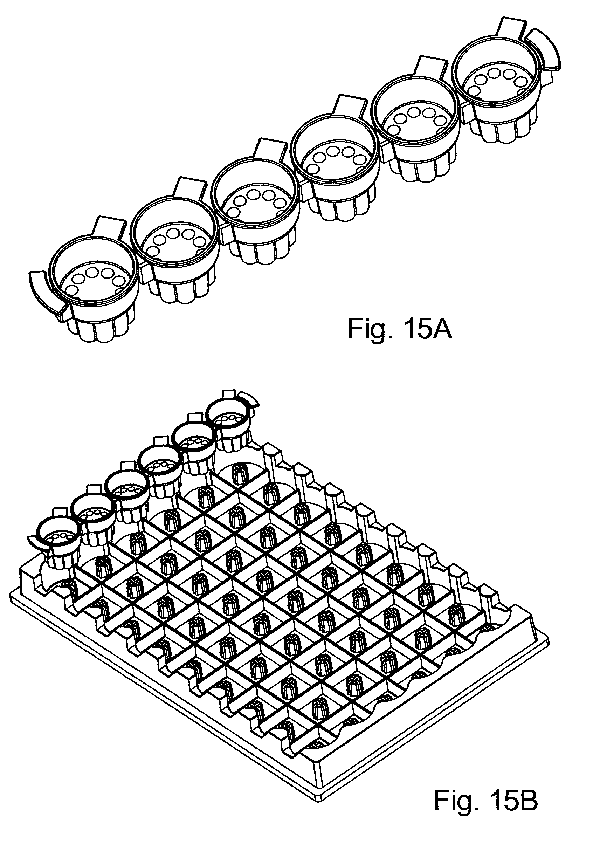

Fig. 15A shows in greater detail a strip of six sample wells. According to the preferred embodiment the sample wells in a strip can be separated or otherwise broken apart. According to an embodiment the sample plate or strip can be separated or divided up into single sample wells. Fig. 15B shows a strip of six sample wells being loaded into a plate frame.

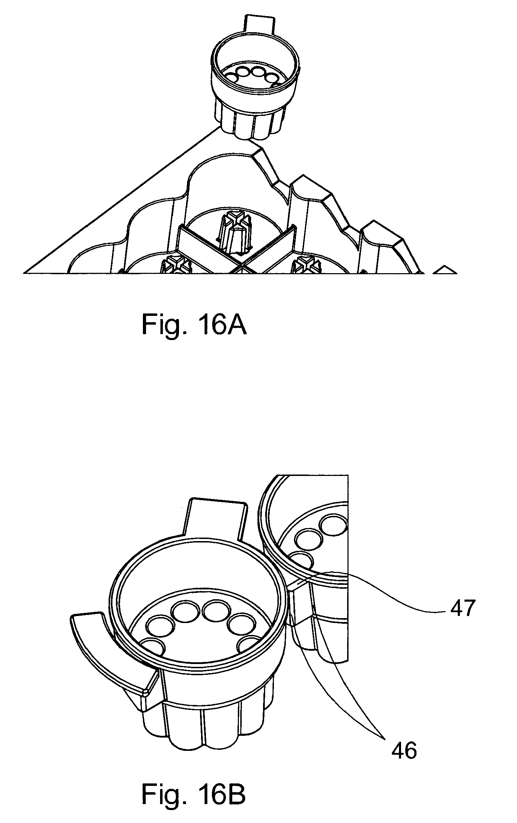

Fig. 16A shows a single sample well (which has been separated from a strip of sample wells) being loaded into a plate frame. The sample wells preferably comprise a female portion which is preferably arranged to engage or interlock with a male portion which is preferably provided on the base of the plate frame. The sample plate or sample strip is preferably arranged to be firmly secured and fixed to the plate frame when loaded onto the plate frame.

Fig. 16B shows in greater detail two sample wells which are connected by a break-apart feature 47. The break-apart feature 47 preferably allows a user to separate adjacent sample wells. According to an embodiment sample wells may be separated from each other but may still be placed next to each other on the plate frame without interfering with each other. The break-apart feature 47 preferably comprises one, two or more than two break points 46. According to an embodiment the connecting piece 47 between two sample wells may be separated from a sample well at a first break point 46. The connecting piece 47 may then be broken off or otherwise removed from the single sample well that it is attached to by breaking the connecting piece 47 from the sample well at a second break point 46.

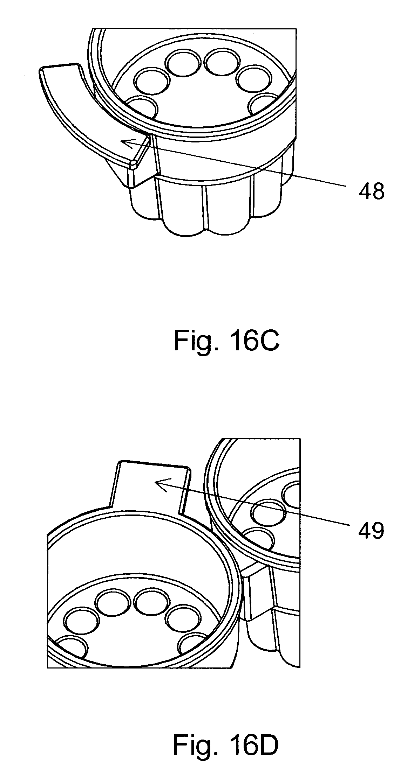

Fig. 16C shows a sample well having an end break-apart feature 48. The end break-apart feature 48 allows the end wells to be used singly in the plate frame without interfering with another sample well. The end break-apart feature 48 provides something for a user to hold in order to remove a strip of sample wells or a single sample well from the plate frame.

Fig. 16D shows a sample well having an ID and orientation tab 49. The tab 49 allows an identifier to be printed onto the tab 49 or to be otherwise attached to the tab 49. The identifier may comprise a 2D or 3D barcode and/or human readable text. The tab 49 preferably assists a user to orientate a sample well when a single sample well is used by aligning with features in the plate frame and/or on other sample wells.

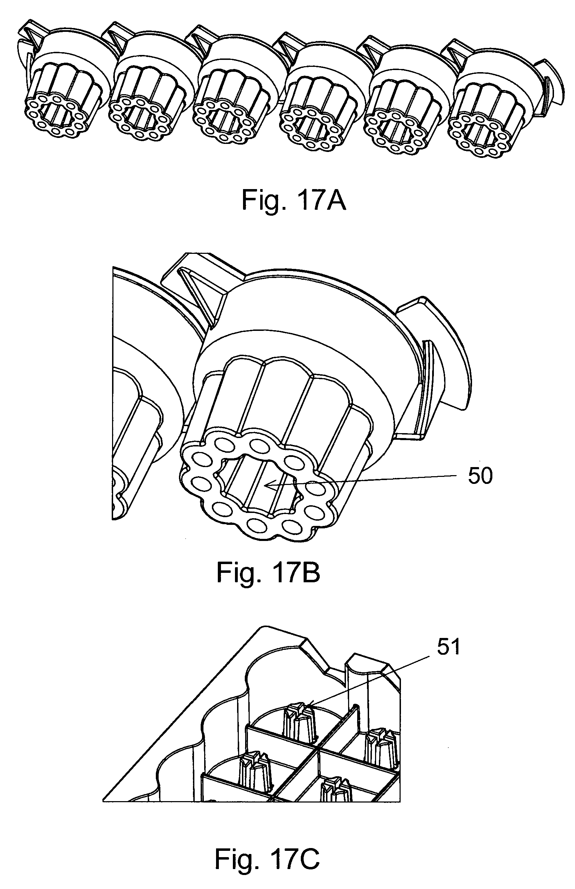

Fig. 17A shows the underneath of a strip of sample wells and shows that according to the preferred embodiment each sample well comprises ten bores or recesses in which a reagent bead is preferably inserted in use. The base or underside of each sample well preferably also comprises a female portion which is preferably arranged to be mated, in use, with a male portion which is provided in the base of the plate frame.

Fig. 17B shows in greater detail a female alignment and retaining feature 50 which helps to align a strip of sample wells with a plate frame. Fig. 17C shows a corresponding male alignment and retaining feature 51 which is preferably provided in the base of the plate frame. The male portion 51 may according to an embodiment comprise a plurality of flexible projections which are preferably deformed inwards as a sample well is located over the male portion 51. The projections on the plate frame preferably move or close together ensuring that the sample well is kept in place without having to apply undue force either to mount or fix a sample well onto the plate frame and/or to demount a sample well from the plate frame.

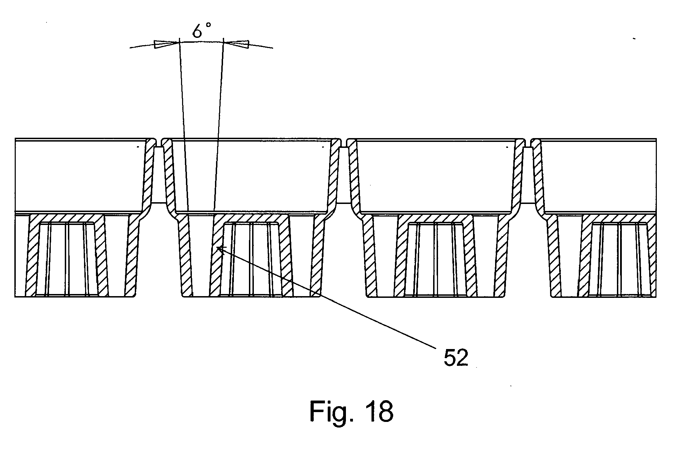

Fig. 18 shows a cross-sectional view of a strip of sample wells and shows that according to the preferred embodiment the sample wells preferably have a plurality of tapered bores 52. The tapered bores 52 preferably act as pockets into which a reagent bead may be inserted in use. The angle of the taper is preferably 6.0°.

Although various embodiments described above have focussed upon reagent beads which are coated with a biomolecule for use in an Immunoassay or ELISA procedure, the present invention equally applies to reagent beads which comprise or which are otherwise coated with a nucleic acid sequence and which are used as a hybridization probe for the detection of DNA or RNA sequences which are complementary to those provided on the reagent beads. As will be understood by those skilled in the art, the hybridization probe will be inactive until hybridization, at which point there is a conformational change and the molecule complex becomes active and will then fluoresce under UV light. Therefore, all the various embodiments described above and all the various aspects of the embodiments described above apply equally to the use of reagent beads comprising or which are otherwise coated with a DNA or RNA sequence (or other nucleotide sequence) for use as a hybridization probe to detect complementary DNA or RNA sequences.
Although the present invention has been described with reference to preferred embodiments, it will be understood by those skilled in the art that various changes in form and detail may be made without departing from the scope of the invention as set forth in the accompanying claims.

## Claims

1. A sample plate comprising one or more sample wells (19), wherein one or more of said sample wells (19) comprise:
a base portion;
a plurality of pockets or recesses (21) provided in said base portion, wherein said plurality of pockets or recesses (21) each comprise a bore having a tapered section; and
a reagent bead or microsphere (20A,20B) inserted or located in each of said bores;
**characterised in that**:
the opening to each bore and/or the cross-sectional shape of each bore is circular; and
wherein each said reagent bead or microsphere (20A,20B) inserted within one of said bores forms a press fit with said tapered section of said bore.

2. A sample plate as claimed in claim 1, wherein said reagent beads or microspheres (20A,20B) have a diameter selected from the group consisting of: (i) 0.5-1.0 mm; (ii) 1.0-1.5 mm; (iii) 1.5-2.0 mm; (iv) 2.0-2.5 mm; (v) 2.5-3.0 mm; (vi) 3.0-3.5 mm; (vii) 3.5-4.0 mm; (viii) 4.0-4.5 mm; (ix) 4.5-5.0 mm; and (x) > 5.0 mm.

3. A sample plate as claimed claim 1 or 2, wherein one or more of said sample wells (19) are interconnected to one or more other sample wells by one or more frangible regions or connections (47) so that said sample plate can be separated by a user into a plurality of smaller sample plates.

4. A sample plate as claimed in claim 1, 2 or 3, wherein said sample plate comprises an Immunoassay sample plate.

5. A sample plate as claimed in any preceding claim, wherein at least some or substantially all of said reagent beads or microspheres (20A,20B) carry, comprise or are otherwise coated with a reagent, wherein said reagent is arranged and adapted to assay for an analyte of interest in a sample liquid.

6. A sample plate as claimed in claim 1, 2 or 3, wherein said sample plate comprises a hybridization probe for detecting the presence of complementary DNA or RNA samples.

7. A sample plate as claimed in claim 1, 2, 3 or 6, wherein at least some or substantially all of said reagent beads or microspheres (20A,20B) carry, comprise or are otherwise coated with a nucleic acid probe, wherein said nucleic acid probe is arranged and adapted to hybridize with single-stranded nucleic acid, DNA or RNA.

8. A method of using a sample plate to analyse a sample for multiple analytes comprising:
providing a sample plate as claimed in any of claims 1-7; and
adding a sample to said sample well (19).

9. A kit for performing an Enzyme Linked ImmunoSorbent Assay (ELISA) procedure comprising:
one or more sample plates as claimed in any of claims 1-7;
wherein said reagent beads or microspheres (20A,20B) are coated with a reagent comprising an antibody, an antigen or another biomolecule.

10. A kit for performing a nucleic acid probe procedure comprising:
one or more sample plates as claimed in any of claims 1-7;
wherein said reagent beads or microspheres (20A,20B) are coated with a DNA or RNA sequence.

11. A method of manufacturing a sample plate comprising:
providing a sample plate comprising one or more sample wells (19) each having a base portion;
forming a plurality of pockets or recesses (21) in said one or more base portions, wherein said plurality of pockets or recesses (21) each comprise a bore having a tapered section, and wherein the opening to each bore and/or the cross-sectional shape of each bore is circular; and
inserting or locating a reagent bead or microsphere (20A,20B) in each of said bores;
wherein each said reagent bead or microsphere (20A,20B) inserted within one of said bores forms a press fit with said tapered section of said bore.

## Patentansprüche

1. Probenplatte, umfassend eine oder mehrere Probenvertiefungen (19), wobei eine oder mehrere der Probenvertiefungen (19) Folgendes umfassen:
einen Grundteil;
eine vielzahl von Taschen oder Aussparungen (21), die in dem Grundteil bereitgestellt sind, wobei die Vielzahl von Taschen oder Aussparungen (21) jeweils eine Bohrung mit einem abgeschrägten Abschnitt aufweist; und
in jeder der Bohrungen ein eingeführtes oder angeordnetes Reagenskügelchen oder -mikrokügelchen (20A, 20B);
**dadurch gekennzeichnet, dass**:
die Öffnung jeder Bohrung und/oder die Querschnittform jeder Bohrung kreisförmig ist;
und
wobei jedes der in eine der Bohrungen eingeführten Reagenskügelchen oder -mikrokügelchen (20A, 20B) eine Druckpassung mit dem abgeschrägten Abschnitt der Bohrung bildet.

2. Probenplatte gemäß Anspruch 1, wobei die Reagenskügelchen oder -mikrokügelchen (20A, 20B) einen Durchmesser aufweisen, ausgewählt aus der Gruppe bestehend aus: (i) 0,5-1,0 mm; (ii) 1,0-1,5 mm; (iii) 1,5-2,0 mm; (iv) 2,0-2,5 mm; (v) 2,5-3,0 mm; (vi) 3,0-3,5 mm; (vii) 3,5-4,0 mm; (viii) 4,0-4,5 mm; (ix) 4,5-5,0 mm; und (x) > 5,0 mm.

3. Probenplatte gemäß Anspruch 1 oder 2, wobei ein oder mehrere der Probenvertiefungen (19) durch eine oder mehrere zerbrechbare Bereiche oder Verbindungen (47) mit einer oder mehreren anderen Probenvertiefungen verbunden sind, so dass die Probenplatte von einem Benutzer in eine Vielzahl von kleineren Probenplatten getrennt werden kann.

4. Probenplatte gemäß Anspruch 1, 2 oder 3, wobei die Probenplatte eine Immunassay-Probenplatte umfasst.

5. Probenplatte gemäß einem der vorstehenden Ansprüche, wobei wenigstens einige oder im Wesentlichen alle der Reagenskügelchen oder -mikrokügelchen (20A, 20B) ein Reagens tragen, umfassen oder anderweitig damit überzogen sind, wobei das Reagens angeordnet und ausgelegt ist, auf einen Analyten von Interesse in einer Probenflüssigkeit zu prüfen.

6. Probenplatte gemäß Anspruch 1, 2 oder 3, wobei die Probenplatte eine Tiybridisierungssonde zum Nachweisen des Vorhandenseins von komplementären DNA- oder RNA-Proben umfasst.

7. Probenplatte gemäß Anspruch 1, 2, 3 oder 6, wobei wenigstens einige oder im Wesentlichen alle der Reagenskügelchen oder -mikrokügelchen (20A, 20B) eine Nucleinsäuresonde tragen, umfassen oder anderweitig damit überzogen sind, wobei die Nucleinsäuresonde angeordnet und ausgelegt ist, mit einzelsträngiger Nucleinsäure, DNA oder RNA, zu hybridisieren,

8. Verfahren zum Verwenden einer Probenplatte zum Analysieren einer Probe auf mehrere Analyten, umfassend:
Bereitstellen einer Probenplatte gemäß einem der Ansprüche 1-7; und
Zugeben einer Probe zu der Probenvertiefung (19).

9. Kit zum Durchführen eines Enzyme-Linked-Immunosorbent-Assay(ELISA)-Verfahrens, umfassend:
eine oder mehrere Probenplatten gemäß einem der Ansprüche 1-7;
wobei die Reagenskügelchen oder -mikrokügelchen (20A, 20B) mit einem Reagens, umfassend einen Antikörper, ein Antigen oder ein anderes Biomolekül, überzogen sind.

10. Kit zum Durchführen eines Nucleinsäuresonden-Verfahrens, umfassend:
eine oder mehrere Probenplatten gemäß einem der Ansprüche 1-7;
wobei die Reagenskügelchen oder -mikrokügelchen (20A, 20B) mit einer DNA- oder RNA-Sequenz überzogen sind.

11. Verfahren zum Herstellen einer Probenplatte, umfassend:
Bereitstellen einer Probenplatte, die eine oder mehrere Probenvertiefungen (19) umfasst, die jeweils einen Grundteil aufweisen;
Bilden einer Vielzahl von Taschen oder Aussparungen (21) in dem einen Grundteil oder den mehreren Grundteilen, wobei die Vielzahl von Taschen oder Aussparungen (21) jeweils eine Bohrung mit einem abgeschrägten Abschnitt aufweist und wobei die Öffnung jeder Bohrung und/oder die Querschnittform jeder Bohrung kreisförmig ist; und
Einführen oder Anordnen eines Reagenskügelchens oder -mikrokügelchens (20A, 20B) in jeder der Bohrungen;
wobei jedes der in eine der Bohrungen eingeführten Reagenskügelchen oder -mikrokügelchen (20A, 20B) eine Druckpassung mit dem abgeschrägten Abschnitt der Bohrung bildet.

## Revendications

1. Plaque à échantillons comprenant un ou plusieurs puits à échantillon (19), dans laquelle un ou plusieurs desdits puits à échantillon (19) comprennent :
une partie de base ;
une pluralité de poches ou cavités (21) disposées dans ladite partie de base, ladite pluralité de poches ou de cavités (21) comprenant chacune un trou ayant une section conique ; et
une bille ou microsphère de réactif (20A, 20B) introduite ou logée dans chacun desdits trous ; **caractérisée en ce que** :
l'ouverture vers chaque trou et/ou la forme en section transversale de chaque trou est circulaire ; et dans laquelle chaque dite bille ou microsphère de réactif (20A, 20B) introduite dans l'un desdits trous forme un ajustement serré avec ladite section conique dudit trou.

2. Plaque à échantillons selon la revendication 1, dans laquelle lesdites billes ou microsphères de réactif (20A, 20B) ont un diamètre choisi dans le groupe constitué par :
(i) 0,5-1,0 mm ;
(ii) 1,0-1,5 mm ;
(iii) 1,5-2,0 mm ;
(iv) 2,0-2,5 mm ;
(v) 2,5-3,0 mm ;
(vi) 3,0-3,5 mm ;
(vii) 3,5-4,0 mm ;
(viii) 4,0-4,5 mm ;
(ix) 4,5-5,0 mm et
(x) > 5,0 mm.

3. Plaque à échantillons selon la revendication 1 ou 2, dans laquelle un ou plusieurs desdits puits à échantillon (19) sont reliés à un ou plusieurs autres puits à échantillon par une ou plusieurs régions ou liaisons frangibles (47) afin que ladite plaque à échantillons puisse être séparée par un utilisateur en une pluralité de plaques à échantillons plus petites.

4. Plaque à échantillons selon la revendication 1, 2 ou 3, ladite plaque à échantillons comprenant une plaque à échantillons pour immunoessai.

5. Plaque à échantillons selon l'une quelconque des revendications précédentes, dans laquelle au moins une partie ou pratiquement la totalité desdites billes ou microsphères de réactif (20A, 20B) portent un réactif, comprennent celui-ci ou sont sinon enrobées de celui-ci, dans laquelle ledit réactif est conçu et adapté pour doser un analyte d'intérêt dans un liquide échantillon.

6. Plaque à échantillons selon la revendication 1, 2 ou 3, ladite plaque à échantillons comprenant une sonde d'hybridation pour la détection de la présence d'échantillons d'ADN ou ARN complémentaire.

7. Plaque à échantillons selon la revendication 1, 2, 3 ou 6, dans laquelle au moins une partie ou pratiquement la totalité desdites billes ou microsphères de réactif (20A, 20B) portent une sonde d'acide nucléique, comprennent celle-ci ou sont sinon enrobées de celle-ci, dans laquelle ladite sonde d'acide nucléique est conçue et adaptée pour s'hybrider avec un acide nucléique, ADN ou ARN simple brin.

8. Procédé d'utilisation d'une plaque à échantillons pour analyser un échantillon en ce qui concerne de multiples analytes comprenant :
l'obtention d'une plaque à échantillons selon l'une quelconque des revendications 1-7 ; et
l'ajout d'un échantillon audit puits à échantillon (19).

9. Ensemble permettant la mise en oeuvre d'une procédure de dosage immuno-enzymatique (ELISA) Comprenant :
une ou plusieurs plaques à échantillons selon l'une quelconque des revendications 1-7 ;
dans lequel lesdites billes ou microsphères de réactif (20A, 20B) sont enrobées d'un réactif comprenant un anticorps, un antigène ou une autre biomolécule.

10. Ensemble permettant la mise en oeuvre d'une procédure utilisant une sonde d'acide nucléique comprenant :
une ou plusieurs plaques à échantillons selon l'une quelconque des revendications 1-7 ;
dans lequel lesdites billes ou microsphères de réactif (20A, 20B) sont enrobées d'une séquence d'ADN ou d'ARN.

11. Procédé de fabrication d'une plaque à échantillons comprenant :
l'obtention d'une plaque à échantillons comprenant un ou plusieurs puits à échantillon (19) ayant chacun une partie de base ;
la formation d'une pluralité de poches ou cavités (21) dans ladite ou lesdites parties de base, ladite pluralité de poches ou de cavités (21) comprenant chacune un trou ayant une section conique et l'ouverture vers chaque trou et/ou la forme en section transversale de chaque trou étant circulaire ; et
l'introduction ou le logement d'une bille ou microsphère de réactif (20A, 20B) dans chacun desdits trous ;
dans lequel chaque dite bille ou microsphère de réactif (20A, 20B) introduite dans l'un desdits trous forme un ajustement serré avec ladite section conique dudit trou.
